(19) Europäisches Patentamt
European Patent Office
Office européen des brevets

(11) Publication number: **0 104 070**
**B1**

(12) # EUROPEAN PATENT SPECIFICATION

(45) Date of publication of patent specification: **30.12.86**

(51) Int. Cl.$^4$: **C 07 C 155/00,** C 07 D 317/12, A 01 N 47/20

(21) Application number: **83305464.6**

(22) Date of filing: **16.09.83**

(54) **Fungicidal N-phenylcarbamates.**

(30) Priority: **16.09.82 GB 8226441**
**10.12.82 JP 217338/82**

(43) Date of publication of application:
**28.03.84 Bulletin 84/13**

(45) Publication of the grant of the patent:
**30.12.86 Bulletin 86/52**

(84) Designated Contracting States:
**BE CH DE FR GB IT LI NL**

(56) References cited:

**CHEMICAL ABSTRACTS, Vol. 26, No. 6, March 20, 1932, Pages 1586-1587, COLUMBUS OHIO (US). D. W. BROWNE et al.: "Inhibitory effect of substituents in chemical reactions. II. Reactivity of the isothiocyano group in substituted arylthiocarbimedes".**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Takahashi, Junya**
**4-2-303, Ryodo-cho**
**Hishinomiya Hyogo (JP)**
Inventor: **Oguri, Yukio**
**10-3-349, Sonehigasdhi-machi 2-chome**
**Toyonaka Osaka (JP)**
Inventor: **Kato, Toshiro**
**8-D-410, Sakasedai 1-chome**
**Takarazuka Hyogo (JP)**
Inventor: **Yamamoto, Shigeo**
**2-16, Koda 2-chome**
**Ikeda Osaka (JP)**
Inventor: **Noguchi, Hiroshi**
**10-3-318, Sonehigashi-machi 2-chome**
**Toyonaka Osaka (JP)**
Inventor: **Kamoshita, Katsuzo**
**3-11, Kofudai 2-chome**
**Toyono-cho Toyona-gun Osaka (JP)**

(74) Representative: **Allard, Susan Joyce et al**
**BOULT, WADE & TENNANT 27 Furnival street**
**London EC4A 1PQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 104 070 B1

**Description**

This invention relates to fungicidal N-phenylcarbamates.

Benzimidazole and thiophanate fungicides such as Benomyl (methyl 1-butylcarbamoyl)benzimidazol-2-ylcarbamate), Fuberidazol (2-(2-furyl)benzimidazole), Thiabendazole (2-(4-thiazolyl)benzimidazole, Carbendazim (methyl benzimidazol-2-ylcarbamate), Thiophanate-methyl (1,2-bis(3-methoxycarbonyl-2-thioureido)benzene), Thiophanate (1,2-bis(3-ethoxycarbonyl-2-thioureido)benzene), 2-(O,S-dimethylphosphorylamino)-1-(3′-methoxycarbonyl-2′-thioureido)benzene and 2-(O,O-dimethylthiophosphorylamino)-1-(3′-methoxycarbonyl-2′-thioureido)benzene are known to show an excellent fungicidal activity against various plant pathogenic fungi, and they have been widely used as agricultural fungicides since 1970. However, their continuous application over a long period of time provides phytopathogenic fungi with tolerance to them, whereby there plant disease-preventive effect is lowered. Further, the fungi which have gained tolerance to certain kinds of benzimidazole or thiophanate fungicides also show considerable tolerance to some other kinds of benzimidazole or thiophanate fungicides. Thus, they are apt to acquire a cross-tolerance. Therefore, if any significant decrease of their plant disease-preventive effect in certain fields is observed, their application such fields has to be discontinued. However, it is often observed that the density of drug-resistant organisms does not decrease even long after the discontinuation of the use. Although other kinds of fungicides have to be employed in such cases, only a few are as effective as benzimidazole or thiophanate fungicides in controlling various phytopathogenic fungi.

Cyclic imide fungicides such as Procymidone (3-(3′,5′-dichlorophenyl)-1,2-dimethylcyclopropane-1,2-dicarboximide), Iprodione (3-(3′,5′-dichlorophenyl)-1-isopropylcarbamoylimidazolidine-2,4-dione), Vinclozolin (3-(3′,5′-(dichlorophenyl)-5-methyl-5-vinyloxazolidine-2,4-dione), ethyl (RS)-3-(3′,5′-dichlorophenyl)-5-methyl-2,4-dioxooxazolidine-5-carboxylate, etc, which are effective against various plant diseases, particularly those caused by *Botrytis cinerea,* , have the same defects as previously explained with respect to the benzimidazole and thiophanate fungicides.

In C. R. Acad. Sc. Paris, t. 289, S'erie D, pages 691—693 (1979), it is describe that such herbicides as Barban (4-chloro-2-butynyl N-(3-chlorophenyl)carbamate), Chlorobufam (1-methyl-2-propynyl N-(3-chlorophenyl)carbamate), Chlorpropham (isopropyl N-(3-chlorophenyl)carbamate), and Propham (isopropyl N-phenylcarbamate) exhibit a fungicidal activity against certain organisms which show a tolerance to some benzimidazole and thiophanate fungicides. However, their fungicidal activity against the drug-resistant fungi is not very strong, and hence, in practice they can not be used as fungicides.

We have now found that N-phenylcarbamates of the formula:

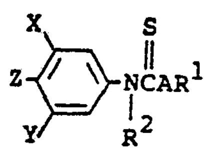

(I)

wherein X and Y are the same or different and each represent a halogen atom, a lower alkyl group, a lower alkenyl group, a lower cyanoalkenyl group, a lower alkynyl group, a lower alkoxy group, a cyano group, a lower alkyl group substituted with at least sone halogen atom, hydroxyl or cyano group, or a group of the formula:

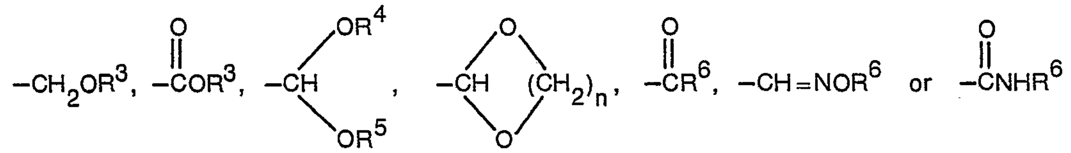

in which $R^3$ is a lower alkyl group a lower alkenyl group, a lower alkynyl group or a lower haloalkyl group, $R^4$ and $R^5$ are the same or different and each represent a lower alkyl group, $R^6$ is a hydrogen atom or a lower alkyl group and n is 2, 3 or 4; Z is a hydrogen agom, a fluorine atom or a group of the formula: $—OR^7$ in which $R^7$ is a lower alkyl group, a lower alkenyl group, a lower alkynyl group or a lower alkyl group substituted with at least one halogen atom, lower alkoxy or lower cycloalkyl group; A is an oxygen atom or a sulfur atom; $R^1$ is a $C_1—C_8$ alkyl group, a $C_3—C_8$ alkenyl group, a $C_3—C_8$ alkynyl group, a lower cycloalkyl group, a lower haloalkenyl group, a lower haloalkynyl group or a lower alkyl group substituted with at least one halogen atom, cyano, lower alkoxy, lower alkenyloxy, lower haloalkoxy, phenoxy, lower aralkyloxy, phenyl or lower cycloalkyl group; and $R^2$ is a hydrogen atom, a cyano group, a lower alkyl group, a lower alkenyl group, a lower alkynyl group, a lower cycloalkyl group, a lower haloalkenyl group, a lower alkyl group substituted with at least one halogen atom, cyano, lower alkoxy, lower alkoxy, lower alkoxycarbonyl, lower alkylcarbonyl, phenoxycarbonyl (the phenoxy group optionally being substituted with at least one halogen atom, cyano, nitro, trifluoromethyl, lower alkyl or lower alkoxy group), lower cycloalkyl, phenoxy (the phenoxy group optionally being substituted with at least one halogen atom and/or at least one alkyl

group) or heteroaryl group (the heteroaryl group optionally being substituted with at least one halogen atom and/or at least one alkyl group), a phenyl group, a phenyl group substituted with at least one halogen atom, cyano, nitro, trifluoromethyl, lower alkyl or lower alkoxy group, an aralkyl group (the aralkyl group optionally being substituted with at least one halogen atom and/or at least one alkyl group) or a group of the formula: $—COR^8$ or $—Si(R^9)_3$ in which $R^8$ is a lower alkyl group, a lower alkenyl group, a lower alkynyl group, a lower cycloalkyl group, a lower haloalkenyl group, a lower alkyl group substituted with at least one halogen atom, cyano, lower alkoxy, lower cycloalkyl or phenoxy group (the phenoxy group optionally being substituted with at least one halogen atom and/or at least one alkyl group), a phenyl group, a phenyl group substituted with at least one halogen atom, cyano, nitro, trifluoromethyl, lower alkyl or lower alkoxy group, or an aralkyl group (the aralkyl group optionally being substituted with at least one halogen atom and/or at least one alkyl group) and $R^9$ is a lower alkyl group, a lower alkoxy group, a halogen atom or a group of the formula: $—SR^{10}$ in which $R^{10}$ is a lower alkyl group, a lower alkoxycarbonyl group or a phenyl group (the phenyl group optionally being substituted with at least one halogen atom, cyano, nitro, trifluoromethyl, lower alkyl or lower alkoxy group), show an excellent fungicidal activity against plant pathogenic fungi which have developed resistance to benzimidazole, thiophanate and/or cyclic imide fungicides. It is notable that their fungicidal potency against the organisms tolerant to benzimidazole, thiophanate and/or cyclic imide fungicides (hereinafter referred to as "drug-resistant fungi" or "drug-resistant strains") is much higher than that against organisms sensitive to benzimidazole, thiophanate and/or cyclic imide fungicides (hereinafter referred to as "drug-sensitive fungi" or "drug-sensitive strains").

By the term "lower" used herein in connection with organic radicals or compounds is meant that such radicals or compounds each have not more than 6 carbon atoms. Likewise, by the term "heteroaryl" used herein in connection with organic radicals or compounds is meant 5-membered heterocycles, condensed 5-membered heterocycles or 6-membered heterocycles such as furyl, thienyl, pyrrolyl, pyrazolyl, imidazolyl, 1,2,3-triazolyl, 1,2,4-triazolyl, oxazolyl, isoxazolyl, thiazolyl, isothiazolyl, benzofuranyl, benzothienyl, indolyl, benzimidazolyl, indazolyl, benzoxazolyl, 1,2-benzisoxazolyl, benzothiazolyl, pyridinyl, pyridazinyl, pyrimidinyl and pyrazinyl. By the term "halogen" used herein is meant fluorine, chlorine, bromine or iodine.

Thus, the present invention provides a fungicidal composition which comprises, as an active ingredient, a fungicidally effective amount of the N-phenylcarbamate (I) together with an inert carrier or diluent. It also provides a combination comprising as active ingredients the N-phenylcarbamate (I) together with a benzimidazole, thiophanate and/or a cyclic imide fungicide, which is fungicidally effective against not only drug-sensitive fungi but also drug-resistant fungi, and hence particularly effective for the prevention of plant diseases. It also provides a method of controlling plant pathogenic fungi including drug-resistant strains and drug-sensitive strains by applying a fungicidally effective amount of the N-phenylcarbamate (I) to plant pathogenic fungi. It further provides novel N-phenylcarbamates which are presented by the formula (I) wherein X, Y, Z, A, $R^1$ and $R^2$ are each as defined above, with the provisos that when X is a chlorine atom, Z and $R^2$ are each a hydrogen atom, A is an oxygen atom and $R^1$ is an ethyl group, Y can not be a chlorine atom, a methyl group or a methoxy group and that when Z and $R^2$ are each a hydrogen atom, A is an oxygen atom and $R^1$ is an ethyl group, X and Y can not be simultaneously a methyl group or a methoxy group.

Typical examples of the N-phenylcarbamates of the formula (I) are given in Table 1.

TABLE 1

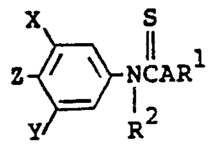

| X | Y | Z | A | $R^1$ | $R^2$ |
|---|---|---|---|---|---|
| $-Cl$ | $-CH_3$ | $-H$ | O | $-CH_3$ | $-H$ |
| $-Cl$ | $-Cl$ | $-F$ | O | $-CH(CH_3)-CH_2OCH_3$ | $-H$ |
| $-Cl$ | $-Cl$ | $-OC_2H_5$ | S | $-CH(CH_3)-CH_3$ | $-H$ |
| $-F$ | $-Br$ | $-OC_2H_5$ | O | $-CH(CH_3)-CH_3$ | $-H$ |
| $-CHBrCH_2Br$ | $-OC_2H_5$ | $-OC_2H_5$ | O | $-CH(CH_3)-CH_3$ | $-H$ |
| $-CH_2OH$ | $-CH_2OCH_3$ | $-OC_2H_5$ | O | $-CH(CH_3)-CH_3$ | $-H$ |
| $-Cl$ | $-Cl$ | $-OC_2H_5$ | O | $-CH_2CH{=}CHCH_3$ | $-H$ |
| $-Cl$ | $-Cl$ | $-OC_2H_5$ | O | $-CH_2C{\equiv}CCH_3$ | $-H$ |
| $-Cl$ | $-Cl$ | $-OC_2H_5$ | O | $-CH_2CH_2C{\equiv}N$ | $-H$ |
| $-CH_2C{\equiv}N$ | $-OC_2H_5$ | $-OC_2H_5$ | O | $-CH(CH_3)-CH_3$ | $-H$ |
| $-CH_2OCH_2CH{=}CH_2$ | $-OC_2H_5$ | $-OC_2H_5$ | O | $-CH(CH_3)-CH_3$ | $-H$ |
| $-CH_2OCH_2C{\equiv}CH$ | $-OC_2H_5$ | $-OC_2H_5$ | O | $-CH(CH_3)-CH_3$ | $-H$ |
| $-CH_3$ | $-CH_3$ | $-OC_2H_5$ | O | $-CH(CH_3)-CH_3$ | $-H$ |
| $-CH_3$ | $-CH_3$ | $-OC_2H_5$ | S | $-CH(CH_3)-CH_3$ | $-H$ |
| $-CH_3$ | $-CH_3$ | $-OC_2H_5$ | O | $-CH_2CH_2F$ | $-H$ |

TABLE 1 (continued)

| X | Y | Z | A | $R^1$ | $R^2$ |
|---|---|---|---|---|---|
| $—CH_2OCH_2CH_2F$ | $—OC_2H_5$ | $—OC_2H_5$ | O | $—CH—CH_3$<br>\|<br>$CH_3$ | —H |
| $—C—OCH_2CH_2F$<br>‖<br>O | —Cl | $—OC_2H_5$ | O | $—CH—CH_3$<br>\|<br>$CH_3$ | —H |
| $—C—NH_2$<br>‖<br>O | $—OC_2H_5$ | $—OC_2H_5$ | O | $—CH—CH_3$<br>\|<br>$CH_3$ | —H |
| —Cl | $—CH_2OCH_3$ | $—OC_2H_5$ | O | 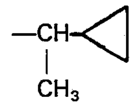 | —H |
| $—CH_3$ | $—OC_2H_5$ | $—OC_2H_5$ | O | $—CH—CH_3$<br>\|<br>$CH_3$ | —H |
| $—CH_3$ | $—OC_2H_5$ | $—OC_2H_5$ | S | $—CH—CH_3$<br>\|<br>$CH_3$ | —H |
| $—C—NHCH_3$<br>‖<br>O | $—OC_2H_5$ | $—OC_2H_5$ | O | $—CH—CH_3$<br>\|<br>$CH_3$ | —H |
| —Cl | $—CH_2OCH_3$ | $—OC_2H_5$ | O | $—CH_3$ | —H |
| —Cl | $—CH_2OCH_3$ | $—OC_2H_5$ | O | $—CH—CH_3$<br>\|<br>$CH_3$ | —H |
| —Cl | $—CH_2OCH_3$ | $—OC_2H_5$ | S | $—CH—CH_3$<br>\|<br>$CH_3$ | —H |
| $—CH_3$ | $—C_2H_5$ | $—OCH_2CH_2CH=CH_2$ | O | $—CH—CH_3$<br>\|<br>$CH_3$ | —H |
| —Cl | $—OC_2H_5$ | $—OCH_2C≡CH$ | O | $—CH—CH_3$<br>\|<br>$CH_3$ | —H |
| $—CH_3$ | $—C_2H_5$ | 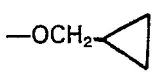 | O | $—CH—CH_3$<br>\|<br>$CH_3$ | —H |
| $—C_2H_5$ | $—C_2H_5$ | $—OCH_2CH_2OCH_3$ | O | $—CH—CH_3$<br>\|<br>$CH_3$ | —H |
| $—CH_3$ | $—CH_2CH=CH_2$ | $—OCH_2CH_2F$ | O | $—CH—CH_3$<br>\|<br>$CH_3$ | —H |
| $—CH=CH_2$ | $—CH_2OCH_3$ | $—OC_2H_5$ | O | $—CH_3$ | —H |

TABLE 1 (continued)

| X | Y | Z | A | $R^1$ | $R^2$ |
|---|---|---|---|---|---|
| $-C\equiv CH$ | $-OC_2H_5$ | $OC_2H_5$ | O | $-CH-CH_3$<br>$\vert$<br>$CH_3$ | $-H$ |
| $-CH(OCH_3)_2$ | $-OC_2H_5$ | $-OC_2H_5$ | O | $-CH_3$ | $-H$ |
| $-CH_3$ | $-C-C_2H_5$<br>$\Vert$<br>$O$ | $-OC_2H_5$ | O | $-CH-CH_3$<br>$\vert$<br>$CH_3$ | $-H$ |
| $-C-OCH_2CH=CH_2$<br>$\Vert$<br>$O$ | $-CH_2OCH_3$ | $-OC_2H_5$ | O | $-CH_3$ | $-H$ |
| $-C-OCH_2C\equiv CH$<br>$\Vert$<br>$O$ | $-CH_2OCH_3$ | $-OC_2H_5$ | O | $-CH_3$ | $-H$ |
| $-C\equiv N$ | $-OC_2H_5$ | $-OC_2H_5$ | O | $-CH-CH_3$<br>$\vert$<br>$CH_3$ | $-H$ |
| $-CH=NOH$ | $-OC_2H_5$ | $-OC_2H_5$ | O | $-CH_3$ | $-H$ |
| $-CH=NOCH_3$ | $-OC_2H_5$ | $-OC_2H_5$ | O | $-CH_3$ | $-H$ |
| $-Cl$ | $-COOCH_3$ | $-OC_2H_5$ | O | $-CH_2CH=CHCH_2Cl$ | $-H$ |
| $-Cl$ | $-COOCH_3$ | $-OC_2H_5$ | O | $-CH_2C\equiv CCH_2Cl$ | $-H$ |
| $-Cl$ | $-COOCH_3$ | $-OC_2H_5$ | O | $-CH_2CH_2OCH_2CH=CH_2$ | $-H$ |
| $-Cl$ | $-COOCH_3$ | $-OC_2H_5$ | O | $-CH_2CH_2OCH_2CH_2Cl$ | $-H$ |
| $-CH_3$ | $-CH_3$ | $-OC_2H_5$ | O | $-CH-C_6H_5$<br>$\vert$<br>$CH_3$ | $-H$ |
| $-Cl$ | $-Cl$ | $-OC_2H_5$ | O | $-CH_2CH_2OC_6H_5$ | $-H$ |
| $-Cl$ | $-COOCH_3$ | $OC_2H_5$ | O | 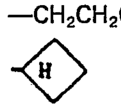 | $-H$ |
| $-Cl$ | $-Cl$ | $-OC_2H_5$ | O | $-CH-CH_2Cl$<br>$\vert$<br>$CH_2OCH_3$ | $-H$ |
| 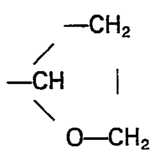 | $-OC_2H_5$ | $-OC_2H_5$ | O | $-CH_3$ | $-H$ |
| $-C-H$<br>$\Vert$<br>$O$ | $-OC_2H_5$ | $-OC_2H_5$ | O | $-CH_3$ | $-H$ |
| $-Cl$ | $-CH_2OCH_3$ | $-OC_2H_5$ | O | $-CH_3$ | $-C_2H_5$ |

TABLE 1 (continued)

| X | Y | Z | A | $R^1$ | $R^2$ |
|---|---|---|---|---|---|
| $-Cl$ | $-CH_2OCH_3$ | $-OC_2H_5$ | O | $-CH_3$ | $-CH_2CH=CH_2$ |
| $-Cl$ | $-CH_2OCH_3$ | $-OC_2H_5$ | O | $-CH_3$ | $-SC_6H_5$ |
| $-Cl$ | $-CH_2OCH_3$ | $-OC_2H_5$ | O | $-CH(CH_3)-CH_3$ | $-C\equiv N$ |
| $-Cl$ | $-CH_2OCH_3$ | $-OC_2H_5$ | O | $-CH_3$ | $-CH_2CH_2C\equiv CH$ |
| $-Cl$ | $-CH_2OCH_3$ | $-OC_2H_5$ | O | $-CH(CH_3)-CH_3$ | $-CH_2CH=CHCH_2Cl$ |
| $-Cl$ | $-CH_2OCH_3$ | $-OC_2H_5$ | O | $-CH_3$ | $-CH_2CH_2Cl$ |
| $-Cl$ | $-OC_2H_5$ | $-OC_2H_5$ | O | $-CH(CH_3)-CH_3$ | $-CH_2CH_2C\equiv N$ |
| $-Cl$ | $-OC_2H_5$ | $-OC_2H_5$ | O | $-CH_3$ | $-CH_2CH_2OCH_3$ |
| $-Cl$ | $-OC_2H_5$ | $-OC_2H_5$ | O | $-CH_3$ | $-CH(CH_3)-COOC_2H_5$ |
| $-Cl$ | $-OC_2H_5$ | $-OC_2H_5$ | O | $-CH(CH_3)-CH_3$ | $-CH_2-C(=O)-CH_3$ |
| $-Cl$ | $-OC_2H_5$ | $-OC_2H_5$ | O | $-CH_3$ | 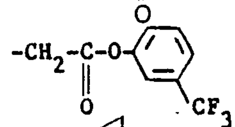 |
| $-CH_3$ | $-OC_2H_5$ | $-OC_2H_5$ | O | $-C_2H_5$ | 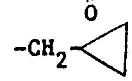 |
| $-CH_3$ | $-OC_2H_5$ | $-OC_2H_5$ | O | $-C_2H_5$ | 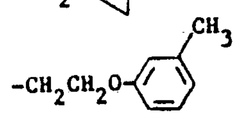 |
| $-CH_3$ | $-OC_2H_5$ | $-OC_2H_5$ | O | $-C_2H_5$ | 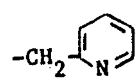 |
| $-CH_3$ | $-OC_2H_5$ | $-OC_2H_5$ | O | $-C_2H_5$ | 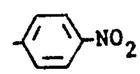 |
| $-CH_3$ | $-OC_2H_5$ | $-OC_2H_5$ | O | $-C_2H_5$ | 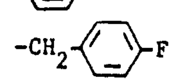 |
| $-CH_3$ | $-CH_2CH_2CH_3$ | $-OC_2H_5$ | O | $-CH_3$ | $-C(=O)-CH_3$ |
| $-CH_3$ | $-CH_2CH_2CH_3$ | $-OC_2H_5$ | O | $-CH_3$ | $-C(=O)-CH=CH_2$ |
| $-CH_3$ | $-CH_2CH_2CH_3$ | $-OC_2H_5$ | O | $-CH_3$ | 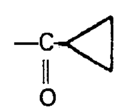 |
| $-CH_3$ | $-CH_2CH_3CH_3$ | $-OC_2H_5$ | O | $-CH_3$ | $-C(=O)-CH_2Cl$ |

TABLE 1 (continued)

| X | Y | Z | A | $R^1$ | $R^2$ |
|---|---|---|---|---|---|
| —Br | $-CH_2OCH_3$ | $-OC_2H_5$ | O | $-CH_3$ | $-\underset{\overset{\|}{O}}{C}-CH_2OCH_3$ |
| —Br | $-CH_2OCH_3$ | $-OC_2H_5$ | O | $-CH_3$ | 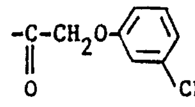 |
| —Br | $-CH_2OCH_3$ | $-OC_2H_5$ | O | $-CH_3$ | 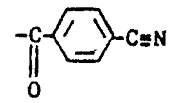 |
| —Br | $-CH_2OCH_3$ | $-OC_2H_5$ | O | $-CH_3$ | 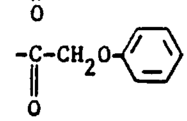 |
| —Br | $-CH_2OCH_3$ | $-OC_2H_5$ | O | $-CH_3$ | $-Si(CH_3)_3$ |

The N-phenylcarbamates of the formula (I) can be prepared by one of the following procedures.

Procedure (a):—

The N-phenylcarbamate of the formula (I) is obtainable by reacting a substituted aniline of the formula:

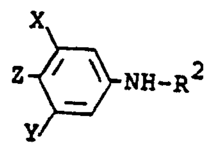

wherein X, Y, Z and $R^2$ are each as defined above, with a chloroformate of the formula:

$$Cl\overset{\overset{S}{\|}}{C}AR^1$$

wherein A and $R^1$ are each as defined above.

The reaction is usually carried out in the presence of an inert solvent (e.g. benzene, toluene, tetrahydrofuran, chloroform, ethyl acetate, dimethylformamide). If desired, the reaction may be performed in the presence of a base (e.g. triethylamine, sodium hydroxide, N,N-diethylaniline) so as to obtain the N-phenylcarbamate (I) in a high yield. The reaction may be carried out at a temperature in the range of from 30 to 120°C, normally within 12 hours.

Procedure (b):—

The N-phenylcarbamate of the formula (I) wherein $R^2$ is a hydrogen atom can be prepared by reacting a substituted phenyl isothiocyanate of the formula:

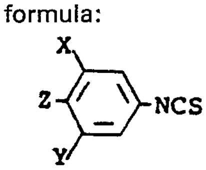

wherein X, Y and Z are each as defined above, with an alcohol or thiol of the formula:

$$HAR^1$$

wherein A and $R^1$ are each as defined above.

The reaction is usually carried out in the presence of an inert solvent (e.g. benzene, toluene, tetrahydrofuran, chloroform, ethyl acetate, dimethylformamide). If desired, the reaction may be performed in the presence of a base (e.g. triethylamine, sodium hydroxide, pyridine). The reaction may be carried out at a temperature in the range of from 0 to 120°C, normally within 12 hours.

Procedure (c):—

The N-phenylcarbamate of the formula (I) wherein at least one of X and Y is a hydroxyiminomethyl group or a lower alkoxyiminomethyl group can be prepared by reacting a benzaldehyde of the formula:

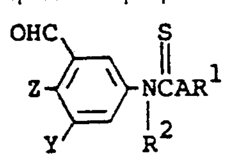

wherein Y, Z, A, $R^1$ and $R^2$ are each as defined above, with hydroxylamine or a lower alkoxyamine.

The reaction is usually carried out by treating the benzaldehyde with at least an equimolar amount of hydroxylamine or the lower alkoxyamine in an inert solvent (e.g. methanol, ethanol) at a temperature in the range of from 20 to 80°C for about 0.5 to 12 hours.

The starting materials employed in the above procedures are known or can be produced by known methods.

The N-phenylcarbamates (I) are fungicidally effective against a wide scope of plant pathogenic fungi, of which examples are as follows: *Podosphaera leucotricha, Venturia inaequalis, Mycosphaerella pomi, Marssonina mali* and *Sclerotinia mali* of apple, *Phyllactinia kakicola* and *Gloeosporium kaki* of persimmon, *Cladosporium carpophilum* and *Phomopsis* sp. of peach, *Cercospora viticola, Uncinula necator, Elsinoe ampelina* and *Glomerella cingulata* of grape, *Cercospora beticola* of sugarbeet, *Cercospora arachidicola* and *Cercospora personata* of peanut, *Erysiphe graminis* f. sp. *hordei, Cercosporella herpotrichoides* and *Fusarium nivale* of barley, *Erysiphe graminis* f. sp. *tritici* of wheat, *Sphaerotheca fuliginea* and *Cladosporium cucumerinum* of cucumber, *Cladosporium fulvum* of tomato, *Corynespora melongenae* of eggplant, *Sphaerotheca humuli, Fusarium oxysporum* f. sp. *fragariae* of strawberry, *Botrytis alli* of onion, *Cercospora apii* of celery, *Phaeoisariopsis griseola* of kidney bean, *Erysiphe cichoracearum* of tobacco, *Diplocarpon rosae* of rose, *Elsinoe fawcetti, Penicillium italicum, Penicillium digitatum* of orange, *Botrytis cinerea* of cucumber, eggplant, tomato, strawberry, pimiento, onion, lettuce, grape, orange, cyclamen, rose or hop, *Sclerotinia sclerotiorum* of cucumber, eggplant, pimiento, lettuce, celery, kidney bean, soybean, azuki bean, potato or sunflower, *Sclerotinia cinerea* of peach or cherry, *Mycosphaerella melonis* of cucumber or melon, etc. Thus, the N-phenylcarbamates (I) are highly effective in controlling the drug-resistant strains of the fungi.

The N-phenylcarbamates (I) are also fungicidally effective against fungi sensitive to known fungicides as well as fungi against which known fungicides are ineffective. Examples of such fungi are *Pyricularia oryzae, Pseudoperonospora cubensis, Plasmopara, viticola, Phytophthora infestans,* etc.

Advantageously, the N-phenylcarbamates possess a low toxicity and do not have a detrimental effect on mammals, fishes, etc. Also, they may be applied to the agricultural field without causing any significant toxicity to important crop plants.

The processes for the preparation of the N-phenylcarbamates (I) are illustrated in the following Examples.

Example 1

Preparation of (3-methoxymethyl-4-ethoxy-5-allyloxycarbonyl)thiocarbanilic acid O-methyl ester according to Procedure (a):—

To a solution of 3-methoxymethyl-4-ethoxy-5-allyloxycarbonylaniline (2.65 g) and N,N-diethylaniline (1.49 g) in 30 ml of toluene was added methyl chlorothioformate (1.20 g) with stirring at 25°C. The resultant mixture was allowed to stand at room temperature for 12 hours, poured into ice-water and extracted with ethyl acetate. The extract was washed with diluted hydrochloric acid and water, dried over magnesium sulfate and concentrated under reduced pressure. The residue was purified by silica gel chromatography using a mixture of toluene and ethyl acetate as the eluent to give (3-methoxymethyl-4-ethoxy-5-allyloxycarbonyl)thiocarbanilic acid O-methyl ester (Compound No. 25) (2.74 g) in a yield of 80.8%. $n_D^{26.5}$ 1.5512.

Example 2

Preparation of (3-chloro-4-ethoxy-5-methoxymethyl)thiocarbanilic acid O-isopropyl ester according to Procedure (b):—

3-Chloro-4-ethoxy-5-methoxymethylphenyl isothiocyanate (2.6 g) was added to an isopropanol solution (50 ml) containing triethylamine (1.0 g). The resultant mixture was allowed to stand at room temperature for 12 hours, poured into ice-water and extracted with ethyl acetate. The extract was washed with water, dried over magnesium sulfate and concentrated under reduced pressure. The residue was purified by silica gel chromatography using a mixture of benzene and tetrahydrofuran as the eluent to give (3-chloro-4-ethoxy-5-methoxymethyl)thiocarbanilic acid O-isopropyl ester (Compound No. 14) (2.9 g) in a yield of 93%. M.P., 92—93°C.

Elementary analysis: Calcd. for $C_{14}H_{20}ClNO_3S$: C, 52.91%; H, 6.34%; N, 4.41%; Cl, 11.15%; S, 10.09%. Found: C, 52.65%; H, 6.35%; N, 4.21%; Cl, 11.44%; S, 10.09%.

Example 3

Preparation of (3,4-diethoxy-5-methoxyiminomethyl)thiocarbanilic acid O-methyl ester according to Procedure (c):—

(3,4-Diethoxy-5-formyl)thiocarbanilic acid O-methyl ester (0.6 g) was dissolved in ethanol (20 ml). To the solution was added an aqueous solution (10 ml) containing sodium hydroxide (0.32 g) and methoxyamine hydrochloride (0.66 g). After being allowed to stand for 12 hours, the reaction mixture was concentrated in vacuo, and the residue was dissolved in ethyl acetate. The resultant solution was washed with water, dried over magnesium sulfate and concentrated under reduced pressure to give (3,4-diethoxy-5-methoxyiminomethyl)thiocarbanlic acid O-methyl ester (Compound No. 29) (0.58 g) in a yield of 87.6%. $n_D^{30.6}$ 1.5657.

According to the above procedures, the N-phenylcarbamates of the formula (I) as shown in Table 2 can be prepared.

TABLE 2

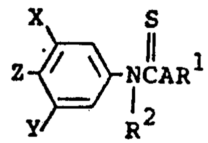

| Compound No. | X | Y | Z | A | $R^1$ | $R^2$ | Physical constant |
|---|---|---|---|---|---|---|---|
| 1 | —Cl | $—CH_3$ | —H | O | $—CH_3$ | —H | M.P. 95—96°C |
| 2 | —Cl | —Cl | —F | O | $—CH—CH_2OCH_3$<br>\|<br>$CH_3$ | —H | M.P. 101—103°C |
| 3 | —Cl | —Cl | $—OC_2H_5$ | S | $—CH—CH_3$<br>\|<br>$CH_3$ | —H | $n_D^{23.5}$ 1.6189 |
| 4 | —Cl | —Cl | $—OC_2H_5$ | O | $—CH_2CH=CHCH_3$ | —H | $n_D^{22.5}$ 1.5526 |
| 5 | —Cl | —Cl | $—OC_2H_5$ | O | $—CH_2C\equiv CCH_3$ | —H | M.P. 158.5—159.5°C |
| 6 | —Cl | —Cl | $—OC_2H_5$ | O | $—CH_2CH_2CN$ | —H | NMR ($CDCl_3$) $\delta_{TMS}$: 1.41 (t, 3H), 2.77 (t, 2H), 4.00 (q, 2H), 4.63 (t, 2H), 7.28 (s, 2H), 7.98 (broad, 1H) |

TABLE 2 (continued)

| Compound No. | X | Y | Z | A | $R^1$ | $R^2$ | Physical constant |
|---|---|---|---|---|---|---|---|
| 7 | $—H_3$ | $—CH_3$ | $—OC_2H_5$ | O | $—CH—CH_3$<br>\|<br>$CH_3$ | —H | M.P. 93—95°C |
| 8 | $—CH_3$ | $—CH_3$ | $—OC_2H_5$ | S<br>\|<br>$CH_3$ | $—CH—CH_3$ | —H | $n_D^{21.4}$ 1.5909 |
| 9 | $—CH_3$ | $—CH_3$ | $—OC_2H_5$ | O | $—CH_2CH_2F$ | —H | $n_D^{24.0}$ 1.5882 |
| 10 | —Cl | $—CH_2OCH_3$ | $—OC_2H_5$ | O | 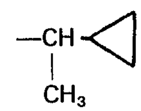 | —H | $n_D^{21.4}$ 1.5759 |
| 11 | $—CH_3$ | $—OC_2H_5$ | $—OC_2H_5$ | O | $—CH—CH_3$<br>\|<br>$CH_3$ | —H | M.P. 75—77° |
| 12 | $—CH_3$ | $—OC_2H_5$ | $—OC_2H_5$ | S | $—CH—CH_3$<br>\|<br>$CH_3$ | —H | $n_D^{21.4}$ 1.5878 |
| 13 | —Cl | $—CH_2OCH_3$ | $—OC_2H_5$ | O | $—CH_3$ | —H | M.P. 88—89°C |
| 14 | —Cl | $—CH_2OCH_3$ | $OC_2H_5$ | O | $—CH—CH_3$<br>\|<br>$CH_3$ | —H | M.P. 92—93°C |

TABLE 2 (continued)

| Compound No. | X | Y | Z | A | $R^1$ | $R^2$ | Physical constant |
|---|---|---|---|---|---|---|---|
| 15 | $-Cl$ | $-CH_2OCH_3$ | $-OC_2H_5$ | S | $-CH-CH_3$<br>\|<br>$CH_3$ | $-H$ | $n_D^{24.2}$ 1.6041 |
| 16 | $-CH_3$ | $-C_2H_5$ | $-OCH_2CH_2CH{=}CH_2$ | O | $-CH-CH_3$<br>\|<br>$CH_3$ | $-H$ | $n_D^{26.0}$ 1.5597 |
| 17 | $-Cl$ | $-OC_2H_5$ | $-OCH_2C{\equiv}CH$ | O | $-CH-CH_3$<br>\|<br>$CH_3$ | $-H$ | $n_D^{26.0}$ 1.5870 |
| 18 | $-CH_3$ | $-C_2H_5$ | $-OCH_2-$ 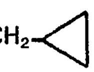 | O | $-CH-CH_3$<br>\|<br>$CH_3$ | $-H$ | NMR ($CDCl_3$) $\delta_{TMS}$: 0.22—1.00 (m, 5H), 1.19 (t, 3H), 1.36 (d, 6H), 2.21 (s, 3H), 2.60 (q, 2H), 3.50 (d, 2H), 5.52 (m, 1H), 6.89 (s, 2H), 8.00 (broad, 1H) |
| 19 | $-C_2H_5$ | $-C_2H_5$ | $-OCH_2CH_2OCH_3$ | O | $-CH-CH_3$<br>\|<br>$CH_3$ | $-H$ | NMR ($CDCl_3$) $\delta_{TMS}$: 1.15 (t, 6H), 1.35 (d, 6H), 2.60 (q, 4H), 3.41 (s, 3H), 3.75 (m, 4H), 5.60 (m, 1H), 6.94 (s, 2), 8.15 (broad, 1H) |

TABLE 2 (continued)

| Compound No. | X | Y | Z | A | $R^1$ | $R^2$ | Physical constant |
|---|---|---|---|---|---|---|---|
| 20 | $-CH_3$ | $-CH_2CH=CH_2$ | $-OCH_2CH_2F$ | O | $-CH-CH_3$ <br> \| <br> $CH_3$ | $-H$ | $n_D^{26.0}$ 1.5642 |
| 21 | $-CH=CH_2$ | $-CH_2OCH_3$ | $-OC_2H_5$ | O | $-CH_3$ | $-H$ | $n_D^{31.5}$ 1.5913 |
| 22 | $-C\equiv CH$ | $-OC_2H_5$ | $-OC_2H_5$ | O | $-CH-CH_3$ <br> \| <br> $CH_3$ | $-H$ | NMR ($CDCl_3$) $\delta_{TMS}$: 1.35 (d, 6H), 1.20 —1.60 (m, 6H), 3.12 (s, 1H), 3.95 (q, 2H), 4.05 (q, 2H), 5.50 (m, 1H), 2H), 5.50 (m, 1H), 6.75 (d, 1H), 6.90 (d, 1H), 7.90 (broad, 1H) |
| 23 | $-CH(OCH_3)_2$ | $-OC_2H_5$ | $-OC_2H_5$ | O | $-CH_3$ | $-H$ | $n_D^{30.6}$ 1.5412 |
| 24 | $-CH_3$ | $-C-C_2H_5$ <br> ‖ <br> O | $-OC_2H_5$ | O | $-CH-CH_3$ <br> \| <br> $CH_3$ | $-H$ | $n_D^{28.0}$ 1.5761 |
| 25 | $-C-OCH_2CH=CH_2$ <br> ‖ <br> O | $-CH_2OCH_3$ | $-OC_2H_5$ | O | $-CH_3$ | $-H$ | $n_D^{26.5}$ 1.5512 |
| 26 | $-C-OCH_2C\equiv CH$ <br> ‖ | $-CH_2OCH_3$ | $-OC_2H_5$ | O | $-CH_3$ | $-H$ | $n_D^{28.0}$ 1.5605 |

TABLE 2 (continued)

| Compound No. | X | Y | Z | A | $R^1$ | $R^2$ | Physical constant |
|---|---|---|---|---|---|---|---|
| 27 | —C≡N | $—OC_2H_5$ | $—OC_2H_5$ | O | $—CH—CH_3$<br>\|<br>$CH_3$ | —H | $n_D^{28.0}$ 1.5812 |
| 28 | —CH=NOH | $—OC_2H_5$ | $—OC_2H_5$ | O | $—CH_3$ | —H | NMR ($CDCl_3$) $\delta_{TMS}$: 1.35 (t, 3H), 1.42 (t, 3H), 4.00 (q, 2H), 4.05 (q, 2H), 4.09 (s, 3H), 7.00 (broad, 1H), 7.20 (2H), 8.49 (s, 1H), 9.03 (broad, 1H) |
| 29 | $—CH=NOCH_3$ | $—OC_2H_5$ | $—OC_2H_5$ | O | $—CH_3$ | —H | $n_D^{30.6}$ 1.5657 |
| 30 | —Cl | $—COOCH_3$ | $—OC_2H_5$ | O | $—CH_2CH=CH$<br>\|<br>$CH_2Cl$ | —H | NMR ($CDCl_3$) $\delta_{TMS}$: 1.44 (t, 3H), 3.89 (s, 3H), 4.08 (q, 2H), 4.08 (q, 2H), 4.30—6.80 (m, 6H), 7.60 (d, 1H), 7.78 (d, 1H) |
| 31 | —Cl | $—COOCH_3$ | $—OC_2H_5$ | O | $—CH_2C≡CCH_2Cl$ | —H | M.P. 126—130°C |
| 32 | —Cl | $—COOCH_3$ | $—OC_2H_5$ | O | $—CH_2CH_2$<br>\|<br>$OCH_2CH=CH_2$ | —H | $n_D^{26.0}$ 1.5802 |

TABLE 2 (continued)

| Compound No. | X | Y | Z | A | $R^1$ | $R^2$ | Physical constant |
|---|---|---|---|---|---|---|---|
| 33 | $-Cl$ | $-COOCH_3$ | $-OC_2H_5$ | O | 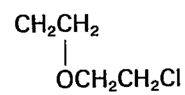 | $-H$ | $n_D^{26.0}$ 1.5832 |
| 34 | $-CH_3$ | $-CH_3$ | $-OC_2H_5$ | O | 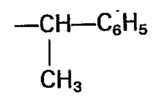 | $-H$ | $n_D^{27.0}$ 1.5800 |
| 35 | $-Cl$ | $-Cl$ | $-OC_2H_5$ | O | $-CH_2CH_2OC_6H_5$ | $-H$ | M.P. 104—106°C |
| 36 | $-Cl$ | $-COOCH_3$ | $-OC_2H_5$ | O | 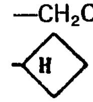 | $-H$ | $n_D^{27.0}$ 1.6080 |
| 37 | $-Cl$ | $-Cl$ | $-OC_2H_5$ | O | 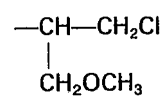 | $-H$ | $n_D^{28.0}$ 1.5627 |
| 38 | 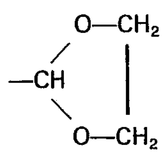 | $-OC_2H_5$ | $-OC_2H_5$ | O | $-CH_3$ | $-H$ | $n_D^{31.5}$ 1.5659 |
| 39 | 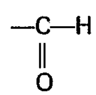 | $-OC_2H_5$ | $-OC_2H_5$ | O | $-CH_3$ | $-H$ | NMR ($CDCl_3$) $\delta_{TMS}$: 1.39 (t, 3H), 1.47 (t, 3H), 4.07 (q, 2H), 4.20 (q, 2H), 4.09 (s, 3H), 7.25 (s, 2H), 8.36 (broad, 1H), 10.40 (s, 1H) |

| Compound No. | X | Y | Z | A | $R^1$ | $R^2$ | Physical constant |
|---|---|---|---|---|---|---|---|
| 40 | —Cl | $—CH_2OCH_3$ | $—OC_2H_5$ | O | $—CH_3$ | $—C_2H_5$ | $n_D^{27.0}$ 1.5567 |
| 41 | —Cl | $—CH_2OCH_3$ | $—OC_2H_5$ | O | $—CH_3$ | $—CH_2CH{=}CH_2$ | $n_D^{27.0}$ 1.5652 |
| 42 | —Cl | $—CH_2OCH_3$ | $—OC_2H_5$ | O | $—CH_3$ | $—SC_6H_5$ | NMR ($CDCl_3$) $\delta_{TMS}$: 1.42 (t, 3H), 3.48 (s, 3H), 4.02 (s, 3H), 4.02 (q, 2H), 4.56 (s, 2H), 7.00—7.30 (m, 7H) |

In the practical usage of the N-phenylcarbamates (I) as fungicides, they may be applied as such or in a formulation form such as dusts, wettable powders, oil sprays, emulsifiable concentrates, tablets, granules, fine granules, aerosols or flowables. Such formulation form can be prepared in a conventional manner by mixing at least one of the N-phenylcarbamates (I) with an appropriate solid or liquid carrier(s) or diluent(s) and, if necessary, an appropriate adjuvant(s) (e.g. surfactants, adherents, dispersants, stabilizers) for improving the dispersibility and other properties of the active ingredient(s).

Examples of the solid carriers or diluents are botanical materials (e.g. flour, tobacco stalk powder, soybean powder, walnut-shell powder, vegetable powder, saw dust, bran, bark powder, cellulose powder, vegetable extract residue), fibrous materials (e.g. paper, corrugated cardboard, old rags), synthetic plastic powders, clays (e.g. kaolin, bentonite, fuller's earth), talcs, other inorganic materials (e.g. pyrophyllite, sericite, pumice, sulfur powder, active carbon) and chemical fertilizers (e.g. ammonium sulfate, ammonium phosphate, ammonium nitrate, urea, ammonium chloride).

Examples of the liquid carriers or diluents are water, alcohols (e.g. methanol, ethanol), ketones (e.g. acetone, methylethylketone), ethers (e.g. diethyl ether, dioxane, cellosolve, tetrahydrofuran), aromatic hydrocarbons (e.g. benzene, toluene, xylene, methyl naphthalene), aliphatic hydrocarbons (e.g. gasoline, kerosene, lamp oil), esters, nitriles, acid amides (e.g. dimethylformamide, dimethylacetamide), halogenated hydrocarbons (e.g. dichloroethane, carbon tetrachloride), etc.

Examples of the surfactants are alkyl sulfuric esters, alkyl sulfonates, alkyaryl sulfonates, polyethylene glycol ethers, polyhydric alcohol esters, etc. Examples of the adherents and dispersants may include casein, gelatin, starch powder, carboxymethyl cellulose, gum arabic, alginic acid, lignin, bentonite, molasses, polyvinyl alcohol, pine oil and agar. As the stabilizers, there may be used PAP (isopropyl acid phosphate mixture), tricresyl phosphate (TCP), tolu oil, epoxydized oil, various surfactants, various fatty acids and their esters, etc.

The foregoing formulations generally contain at least one of the N-phenylcarbamates (I) in a concentration of about 1 to 95% by weight, preferably of 2.0 to 80% by weight. By using the formulations, the N-phenylcarbamates (I) are generally applied in such amounts as 2 to 100 g per 10 are.

When only the drug-resistant strains of phytopathogenic fungi are present, the N-phenylcarbamates (I) may be used alone. However, when the drug-sensitive strains are present together with the drug-resistant strains, their alternate use with benzimidazole, thiophanate and/or cyclic imide fungicides or their combined use with benzimidazole, thiophanate and/or cyclic imide fungicides is favorable. In such alternate or combined use, each active ingredient may be employed as such or in conventional agricultural formulation forms. In case of the combined use, the weight proportion of the N-phenylcarbamate (I) and the benzimidazole thiophanate and/or the cyclic imide fungicide may be from 1:0.1 to 1:10.0.

Typical examples of the benzimidazole, thiophanate and the cyclic imide fungicides are shown in Table 3.

TABLE 3

| Compound | Structure | Name |
|---|---|---|
| A | 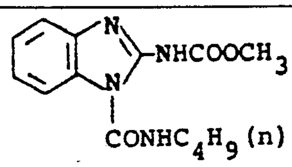 | Methyl 1-(butyl-carbamoyl)benz-imidazole-2-yl-carbamate |
| B | 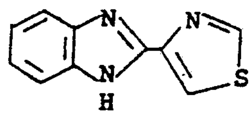 | 2-(4-Thiazolyl)benz-imidazole |
| C | 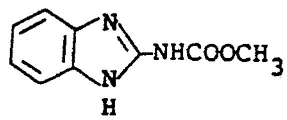 | Methyl benzimidazol-2-ylcarbamate |
| D | 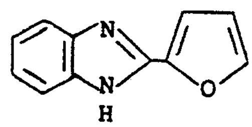 | 2-(2-Furyl)benz-imidazole |
| E | 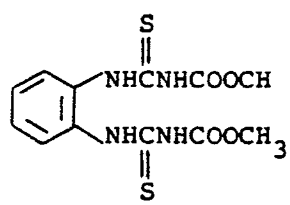 | 1,2-Bis(3-methoxy-carbonyl-2-thio-ureido)benzene |
| F | 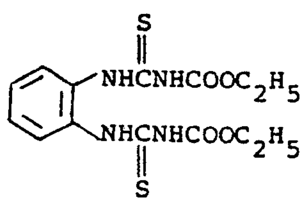 | 1,2-Bis(3-ethoxy-carbonyl-2-thio-ureido)benzene |
| G | 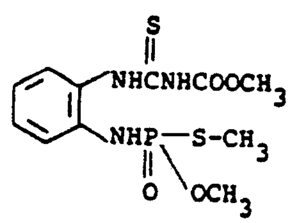 | 2-(O,S-Dimethyl-phosphorylamino)-1-(3′-methoxycarbonyl-2′-thioureido)benzene |
| H | 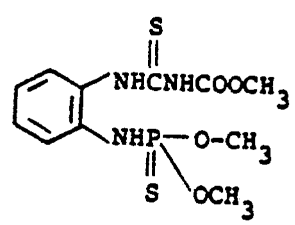 | 2-(O,O-Dimethylthio-phosphorylamino)-1-(3′-methoxycarbonyl-2′-thioureido)benzene |
| I | 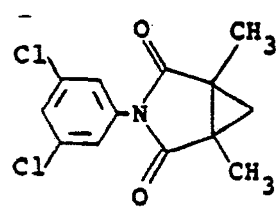 | N-(3′,5′-Dichloro-phenyl)-1,2-dimethyl-cyclopropane-1,2-di-carboximide |

| Compound | Structure | Name |
|---|---|---|
| J | 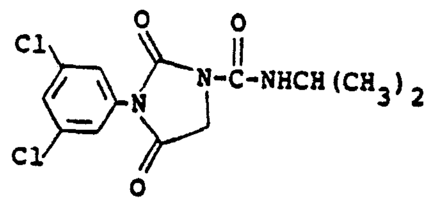 | 3-(3′,5′-Dichlorophenyl)1-isopropylcarbamoylimidazolidine-2,4-dione |
| K | 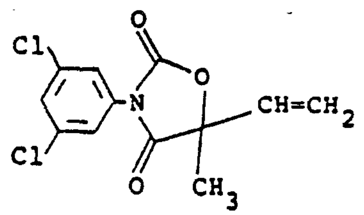 | 3-(3′,5′-Dichlorophenyl)-5-methyl-5-vinyloxazolidine-2,4-dione |
| L | 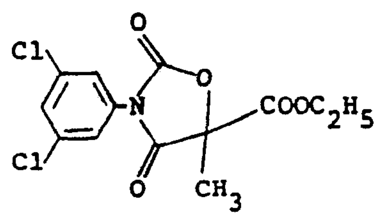 | Ethyl (RS)-3-(3′,5′-dichlorophenyl)-5-methyl-2,4-dioxo-oxazolidine-5-carboxylate |

Besides, the N-phenylcarbamates (I) may be also used in admixture with other fungicides, herbicides, insecticides, miticides, fertilizers, etc.

When the N-phenylcarbamates (I) are used as fungicides, they may be applied in such amounts as 2 to 100 grams per 10 ares. However, this amount may vary depending upon formulation forms, application times, application methods, application sites, diseases, crops and so on, and therefore, they are not limited to said particular amounts.

Some practical embodiments of the fungicidal composition according to the invention are illustratively shown in the following Examples wherein % and part(s) are by weight.

Formulation Example 1

Two parts of Compound No. 9, 88 parts of clay and 10 parts of talc were thoroughly pulverized and mixed together to obtain a dust formulation containing 2% of the active ingredient.

Formulation Example 2

Thirty parts of Compound No. 14, 45 parts of diatomaceous earth, 20 parts of white carbon, 3 parts of sodium laurylsulfate as a wetting agent and 2 parts of calcium ligninsulfonate as a dispersing agent were mixed while being powdered to obtain a wettable powder formulation containing 30% of the active ingredient.

Formulation Example 3

One part of Compound No. 2, 1 part of Compound J, 88 parts of clay and 10 parts of talc are thoroughly pulverized and mixed together to obtain a dust formulation containing 2% of the active ingredient.

Formulation Example 4

Twenty parts of Compound No. 9, 10 parts of Compound K, 45 parts of diatomaceous earth, 20 parts of white carbon, 3 parts of sodium laurylsulfate as a wetting agent and 2 parts of calcium ligninsulfonate as a dispersing agent were mixed while being powdered to obtain a wettable powder formulation containing 30% of the active ingredient.

Formulation Example 5

Two parts of Compound No. 17, 88 parts of clay and 10 parts of talc are thoroughly pulverized and mixed together to obtain a dust formulation containing 2% of the active ingredient.

Formulation Example 6

Ten parts of Compound No. 13, 40 parts of Compound I, 45 parts of diatomaceous earth, 2.5 parts of calcium alkylbenzenesulfonate as a wetting agent and 2.5 parts of calcium ligninsulfonate as a dispersing agent were mixed while being powdered to obtain a wettable powder formulation containing 50% of the active ingredient.

Formulation Example 7

One part of Compound No. 27, 1 part of Compound I, 88 parts of clay and 10 parts of talc are thoroughly pulverized and mixed together to obtain a dust formulation containing 2% of the active ingredient.

Formulation Example 8

Twenty parts of Compound No. 31, 10 parts of Compound J, 45 parts of diatomaceous earth, 20 parts of white carbon, 3 parts of sodium laurylsulfate as a wetting agent and 2 parts of calcium ligninsulfonate as a dispersing agent were mixed while being powdered to obtain a wettable powder formulation containing 30% of the active ingredient.

Formulation Example 9

Fifty parts of Compound No. 15, 45 parts of diatomaceous earth, 2.5 parts of calcium alkylbenzenesulfonate as a wetting agent and 2.5 parts of calcium ligninsulfonate as a dispersing agent were mixed while being powdered to obtain a wettable powder formulation containing 50% of the active ingredient.

Formulation Example 10

Ten parts of Compound No. 5, 80 parts of cyclohexanone and 10 parts of polyoxyethylene alkylaryl ether as an emulsifier were mixed together to obtain an emulsifiable concentrate formulation containing 10% of the active ingredient.

Typical test data indicating the excellent fungicidal activity of the N-phenylcarbamates (I) are shown below. The compounds used for comparison are as follows:

| Compound | Remarks |
|---|---|
| Control (a) 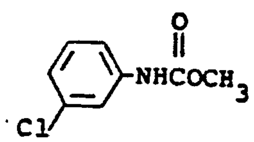 | Synthesized for comparison |
| Control (b) 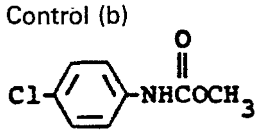 | Synthesized for comparison |
| Control (c) 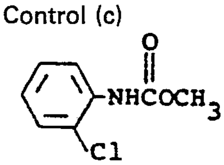 | Synthesized for comparison |
| Control (d) 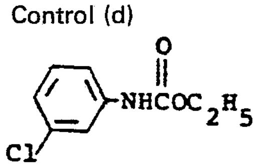 | Synthesized for comparison |
| Control (e) 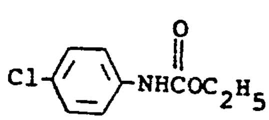 | Synthesized for comparison |

| Compound | Remarks |
|---|---|
| Control (f) 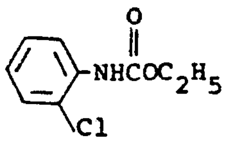 | Synthesized for comparison |
| Control (g) 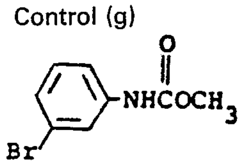 | Synthesized for comparison |
| Control (h) 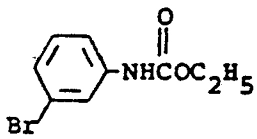 | Synthesized for comparison |
| Control (i) 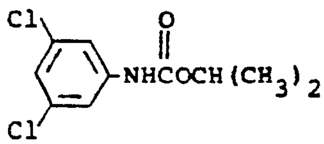 | Agricultural Biological Chemistry, *35,* 1707—1719 (1971) |
| Control (j) 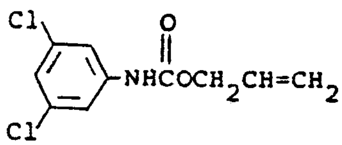 | Agricultural Biological Chemistry, *35,* 1707—1719 (1971) |
| Control (k) 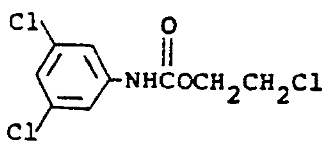 | Agricultural Biological Chemistry, *35,* 1707—1719 (1971) |
| Control (l) 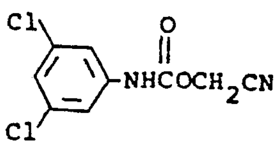 | Agricultural Biological Chemistry, *35,* 1707—1719 (1971) |
| Swep 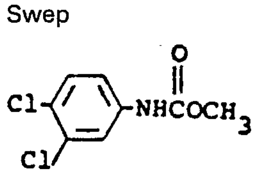 | Commercially available herbicide |
| Chlorpropham 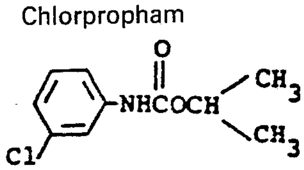 | Commercially available herbicide |

| | |
|---|---|
| Barban 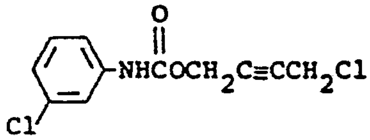 | Commercially available herbicide |
| CEPC 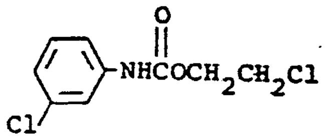 | Commercially available herbicide |
| Propham 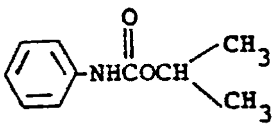 | Commercially available herbicide |
| Chlorbufam 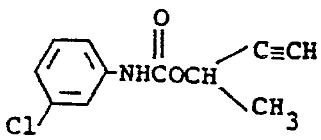 | Commercially available herbicide |
| Benomyl 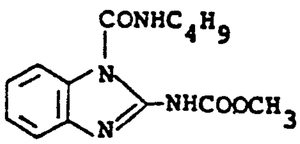 | Commercially available fungicide |
| Thiophanate-methyl 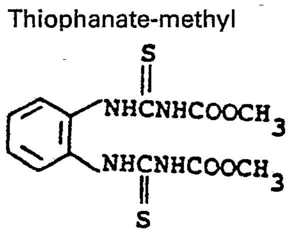 | Commercially available fungicide |
| Carbendazim 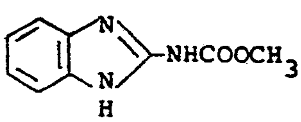 | Commercially available fungicide |
| Thiabendazole 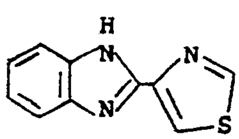 | Commercially available fungicide |
| Edifenphos 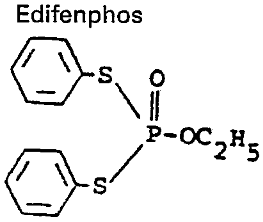 | Commercially available fungicide |

Experiment 1

Protective activity test on powdery mildew of cucumber *(Sphaerotheca fuliginea)*:—

A flower pot of 90 ml volume was filled with sandy soil, and seeds of cucumber (var: Sagami-hanjiro) were sowed therein. Cultivation was carried out in a greenhouse for 8 days. Onto the resulting seedlings having cotyledons, the test compound formulated in emulsifiable concentrate or wettable powder and diluted with water was sprayed at a rate of 10 ml per pot. Then, the seedlings were inoculated with a spore suspension of the drug-resistant or drug-sensitive strain of *Sphaerotheca fuliginea* by spraying and further cultivated in the greenhouse. Ten days thereafter, the infectious state of the plants was observed. The degree of damage was determined in the following manner, and the results are shown in Table 4.

The leaves examined were measured for a percentage of infected area and classified into the corresponding disease indices, 0, 0.5, 1, 2, 4:

| Disease index | Percentage of infected area |
|---|---|
| 0 | No infection |
| 0.5 | Infected area of less than 5% |
| 1 | Infected area of less than 20% |
| 2 | Infected area of less than 50% |
| 4 | Infected area of not less than 50% |

The disease severity was calculated according to the following equation:

$$\text{Disease severity (\%)} = \frac{\Sigma\{(\text{Disease index}) \times (\text{Number of leaves})\}}{4 \times (\text{Total number of leaves examined})} \times 100$$

The prevention value was calculated according to the following equation:

$$\text{Prevention value (\%)} = 100 - \frac{(\text{Disease severity in treated plot})}{(\text{Disease severity in untreated plot})} \times 100$$

Table 4

| Compound No. | Concentration of active ingredient (ppm) | Prevention value when inoculated with drug-resistant strain (%) | Prevention value when inoculated with drug-sensitive strain (%) |
|---|---|---|---|
| 2 | 500 | 100 | 0 |
| 7 | 200 | 100 | 0 |
| 9 | 200 | 100 | 0 |
| 10 | 200 | 100 | 0 |
| 11 | 200 | 100 | 0 |
| 12 | 200 | 100 | 0 |
| 14 | 200 | 100 | 0 |
| Control (a) | 200 | 0 | 0 |
| Control (b) | 200 | 0 | 0 |
| Control (c) | 200 | 0 | 0 |
| Control (d) | 200 | 0 | 0 |
| Control (e) | 200 | 0 | 0 |
| Control (f) | 200 | 0 | 0 |
| Control (g) | 200 | 0 | 0 |
| Control (h) | 200 | 0 | 0 |
| Control (i) | 200 | 0 | 0 |
| Control (j) | 200 | 0 | 0 |
| Control (k) | 200 | 0 | 0 |
| Control (l) | 200 | 0 | 0 |
| Chlorpropham | 200 | 0 | 0 |
| Barban | 200 | 25 | 0 |
| CEPC | 200 | 0 | 0 |
| Propham | 200 | 0 | 0 |
| Chlorbufam | 200 | 0 | 0 |
| Benomyl | 200 | 0 | 100 |
| Thiophanate-methyl | 200 | 0 | 100 |
| Carbendazim | 200 | 0 | 100 |

As understood from the results shown in Table 4, the N-phenylcarbamates (I) of the invention show an excellent preventive effect on the drug-resistant strain but do not show any preventive effect on the tested drug-sensisitve strain. On the contrary, commercially available known fungicides such as Benomyl, Thiophanate-methyl and Carbendazim show a notable controlling effect on the drug-sensitive strain but not on the drug-resistant strain. Other tested compounds structurally similar to the N-phenylcarbamates (I) do not show any fungicidal activity on the drug-sensitive strain and the drug-resistant strain.

Experiment 2

Preventive effect on cercospora leaf spot of sugarbeet *(Cercospora beticola):—*

A flower pot of 90 ml volume was filled with sandy soil, and seeds of sugarbeet (var: Detroit dark red) were sowed therein. Cultivation was carried out in a greenhouse for 20 days. Onto the resulting seedlings, the test compound formulated in emulsifiable concentrate or wettable powder and diluted with water was sprayed at a rate of 10 ml per pot. Then, the seedlings were inoculated with a spore suspension of the drug-resistant or drug-sensitive strain of *Cercospora beticola* by spraying. The pot was covered with a polyvinyl chloride sheet to make a condition of high humidity, and cultivation was continued in the greenhouse for 10 days. The degree of damage was determined in the same manner as in Experiment 1, and the results are shown in Table 5.

Table 5

| Compound No. | Concentration of active ingredient (ppm) | Prevention value when inoculated with drug-resistant strain (%) | Prevention value when inoculated with drug-sensitive strain (%) |
|---|---|---|---|
| 5 | 200 | 94 | 0 |
| 6 | 200 | 97 | 0 |
| 7 | 200 | 100 | 0 |
| 9 | 200 | 100 | 0 |
| 11 | 200 | 98 | 0 |
| 12 | 200 | 100 | 0 |
| 14 | 500 | 100 | 0 |
| 17 | 500 | 100 | 0 |
| Control (a) | 200 | 0 | 0 |
| Control (b) | 200 | 0 | 0 |
| Control (c) | 200 | 0 | 0 |
| Control (d) | 200 | 0 | 0 |
| Control (e) | 200 | 0 | 0 |
| Control (f) | 200 | 0 | 0 |
| Control (g) | 200 | 0 | 0 |
| Control (h) | 200 | 0 | 0 |
| Control (i) | 200 | 0 | 0 |
| Control (j) | 200 | 0 | 0 |
| Control (k) | 200 | 0 | 0 |
| Control (l) | 200 | 0 | 0 |
| Chloropropham | 200 | 0 | 0 |
| Barban | 200 | 34 | 0 |
| CEPC | 200 | 0 | 0 |
| Propham | 200 | 0 | 0 |
| Chlorbufam | 200 | 0 | 0 |
| Benomyl | 200 | 0 | 100 |
| Thiophanate-methyl | 200 | 0 | 100 |
| Carbendazim | 200 | 0 | 100 |

As understood from the results shown in Table 5, the N-phenylcarbamates (I) of the invention show an excellent preventive effect on the drug-resistant strain but do not show any preventive effect on the tested drug-sensitive strain. On the contrary, commercially available known fungicides such as Benomyl, Thiophanate-methyl and Carbendazim show a notable controlling effect on the drug-sensitive strain but not on the drug-resistant strain. Other tested compounds structurally similar to the N-phenylcarbamates (I) do not show any fungicidal activity on the drug-sensitive strain and the drug-resistant strain.

Experiment 3

Preventive effect on scab of pear *(Venturia nashicola)*:—

A plastic pot of 90 ml volume was filled with sandy soil, and seeds of pear (var: Chojuro) were sowed therein. Cultivation was carried out in a greenhouse for 20 days. Onto the resulting seedlings, the test compound formulated in emulsifiable concentrate or wettable powder and diluted with water was sprayed at a rate of 10 ml per pot. Then, the seedlings were inoculated with a spore suspension of the drug-resistant or drug-sensistive strain of *Venturia nashicola* by spraying. The resulting plants were placed at 20°C under a condition of high humidity for 3 days and then at 20°C under irradiation with a fluorescent lamp for 20 days. The degree of damage was determined in the same manner as in Experiment 1, and the results are shown in Table 6.

Table 6

| Compound No. | Concentration of active ingredient (ppm) | Prevention value when inoculated with drug-resistant strain (%) | Prevention value when inoculated with drug-sensitive strain (%) |
|---|---|---|---|
| 1 | 200 | 94 | 0 |
| 11 | 200 | 100 | 0 |
| 12 | 200 | 97 | 0 |
| 27 | 200 | 100 | 0 |
| 31 | 200 | 100 | 0 |
| Benomyl | 200 | 0 | 100 |
| Thiophanate-methyl | 200 | 0 | 100 |

As understood from the results shown in Table 6, the N-phenylcarbamates (I) of the invention show an excellent preventive effect on the drug-resistant strain but do not show any preventive effect on the tested drug-sensitive strain. On the contrary, commercially available known fungicides such as Benomyl and Thiophanate-methyl show a notable controlling effect on the drug-sensitive strain but not on the drug-resistant strain.

Experiment 4

Preventive effect on brown leaf-spot of peanut *(Cercospora arachidicola)*:—

A plastic pot of 90 ml volume was filled with sandy soil, and seeds of peanut (var: Chiba hanryusei) were sowed therein. Cultivation was carried out in a greenhouse for 14 days. Onto the resulting seedlings, the test compound formulated in emulsifiable concentrate or wettable powder and diluted with water was sprayed at a rate of 10 ml per pot. Then, the seedlings were inoculated with a spore suspension of the drug-resistant or drug-sensitive strain of *Cercospora arachidicola* by spraying. The resulting plants were covered with a polyvinyl chloride sheet to make a humide condition and cultivated in the greenhouse for 10 days. The degree of damage was determined in the same manner as in Experiment 1, and the results are shown in Table 7.

Table 7

| Compound No. | Concentration of active ingredient (ppm) | Prevention value when inoculated with drug-resistant strain (%) | Prevention value when inoculated with drug-sensitive strain (%) |
|---|---|---|---|
| 9 | 500 | 100 | 0 |
| 10 | 500 | 100 | 0 |
| 13 | 200 | 97 | 0 |
| 22 | 500 | 100 | 0 |
| 42 | 500 | 100 | 0 |
| Benomyl | 200 | 0 | 100 |
| Thiophanate-methyl | 200 | 0 | 100 |

As understood from the results shown in Table 7, the N-phenylcarbamates (I) of the invention show an excellent preventive effect on the drug-resistant strain but do not show any preventive effect on the tested drug-sensitive strain. On the contrary, commercially available known fungicides such as Benomyl and Thiophanate-methyl show a notable controlling effect on the drug-sensistive strain but not on the drug-resistant strain.

Experiment 5

Preventive effect on gray mold of cucumber *(Botrytis cinerea)*:—

Plastic pots of 90 ml volume was filled with sandy soil, and seeds of cucumber (var: Sagami-hanjiro) were sowed therein. Cultivation was carried out in a greenhouse for 8 days to obtain cucumber seedlings

expanding cotyledons. Onto the resulting seedlings, the test compound formulated in emulsifiable concentrate or wettable powder and diluted with water was sprayed at a rate of 10 ml per pot. After air-drying, the seedlings were inoculated with mycelial disks (5 mm in diameter) of the drug-resistant or drug-sensitive strain of *Botrytis cinerea* by putting them on the leaf surfaces. After the plants were infected by incubating under high humidity at 20°C for 3 days, the rates of disease severity were observed. The degree of damage was determined in the same manner as in Experiment 1, and the results are shown in Table 8.

Table 8

| Compound No. | Concentration of active ingredient (ppm) | Prevention value when inoculated with drug-resistant strain (%) | Prevention value when inoculated with drug-sensitive strain (%) |
|---|---|---|---|
| 1 | 500 | 100 | 0 |
| 2 | 500 | 100 | 0 |
| 3 | 500 | 100 | 0 |
| 4 | 500 | 100 | 0 |
| 5 | 500 | 100 | 0 |
| 6 | 200 | 100 | 0 |
| 7 | 200 | 100 | 0 |
| 8 | 200 | 100 | 0 |
| 9 | 200 | 100 | 0 |
| 10 | 200 | 100 | 0 |
| 11 | 200 | 100 | 0 |
| 12 | 200 | 100 | 0 |
| 13 | 200 | 100 | 0 |
| 14 | 200 | 100 | 0 |
| 15 | 200 | 100 | 0 |
| 16 | 500 | 100 | 0 |
| 17 | 200 | 100 | 0 |
| 18 | 500 | 100 | 0 |
| 19 | 500 | 100 | 0 |
| 20 | 500 | 100 | 0 |
| 21 | 500 | 100 | 0 |
| 22 | 200 | 100 | 0 |
| 23 | 500 | 100 | 0 |
| 24 | 500 | 100 | 0 |
| 25 | 500 | 100 | 0 |
| 26 | 500 | 100 | 0 |
| 27 | 200 | 100 | 0 |
| 28 | 500 | 100 | 0 |
| 29 | 500 | 100 | 0 |
| 30 | 500 | 100 | 0 |
| 31 | 200 | 100 | 0 |
| 32 | 500 | 100 | 0 |
| 33 | 500 | 100 | 0 |
| 34 | 500 | 100 | 0 |
| 35 | 500 | 100 | 0 |
| 36 | 500 | 100 | 0 |
| 37 | 500 | 100 | 0 |
| 38 | 500 | 100 | 0 |
| 39 | 500 | 100 | 0 |
| 40 | 500 | 100 | 0 |
| 41 | 500 | 100 | 0 |
| 42 | 200 | 100 | 0 |
| Benomyl | 200 | 0 | 100 |
| Thiophanate-methyl | 200 | 0 | 100 |
| Thiabendazole | 200 | 0 | 100 |

As understood from the results shown in Table 8, the N-phenylcarbamates (I) of the invention show an excellent preventive effect on the drug-resistant strain but do not show any preventive effect on the tested

drug-sensitive strain. On the contrary, commercially available known fungicides such as Benomyl, Thiophanate-methyl and Thiabendazole show a notable controlling effect on the drug-sensitive strain but not on the drug-resistant strain.

Experiment 6

Preventive effect on gummy stem blight of cucumber *(Mycosphaerella melonis)*:—

Plastic pots of 90 ml volume was filled with sandy soil, and seeds of cucumber (var: Sagami-hanjiro) were sowed therein. Cultivation was carried out in a greenhouse for 8 days to obtain cucumber seedlings expanding cotyledons. Onto the resulting seedlings, the test compound formulated in emulsifiable concentrate or wettable powder and diluted with water was sprayed at a rate of 10 ml per pot. After air-drying, the seedlings were inoculated with mycelial disks (5 mm in diameter) of the drug-resistant or drug-sensitive strain of *Mycosphaerella melonis* by putting them on the leaf surfaces. After the plants were infected by incubating under high humidity at 25°C for 4 days, the rates of disease severity were observed. The degree of damage was determined in the same manner as in Experiment 1, and the results are shown in Table 9.

Table 9

| Compound No. | Concentration of active ingredient (ppm) | Prevention value when inoculated with drug-resistant strain (%) | Prevention value when inoculated with drug-sensitive strain (%) |
|---|---|---|---|
| 9 | 200 | 94 | 0 |
| 13 | 200 | 100 | 0 |
| 14 | 200 | 100 | 0 |
| Benomyl | 200 | 0 | 100 |
| Thiophanate-methyl | 200 | 0 | 100 |

As understood from the results shown in Table 9, the N-phenylcarbamates (I) of the invention show an excellent preventive effect on the drug-resistant strain but do not show any preventive effect on the tested drug-sensitive strain. On the contrary, commercially available known fungicides such as Benomyl and Thiophanate-methyl show a notable controlling effect on the drug-sensitive strain but not on the drug resistant strain.

Experiment 7

Preventive effect on green mold of orange *(Penicillium italicum)*:—

Fruits of orange (var: Unshu) were washed with water and dried in the air. The fruits were immersed in a solution of the test compound prepared by diluting an emulsifiable concentrate comprising the test compound with water for 1 minute. After drying in the air, the fruits were inoculated with a spore suspension of the drug-resistant or drug-sensitive strain of *Penicillium italicum* by spraying and placed in a room of high humidity for 24 days. The degree of damage was determined in the following manner:

The fruits examined were measured for a percentage of infected area and classified into the corresponding indices, 0, 1, 2, 3, 4, 5:

| Disease index | Percentage of infected area |
|---|---|
| 0 | No infection |
| 1 | Infected area of less than 20% |
| 2 | Infected area of less than 40% |
| 3 | Infected area of less than 60% |
| 4 | Infected area of less than 80% |
| 5 | Infected area of not less than 80% |

Calculation of the degree of damage and the prevention value was made as in Experiment 1.

The results are shown in Table 10.

Table 10

| Compound No. | Concentration of active ingredient (ppm) | Prevention value when inoculated with drug-resistant strain (%) | Prevention value when inoculated with drug-sensitive strain (%) |
|---|---|---|---|
| 4 | 200 | 86 | 0 |
| 9 | 200 | 94 | 0 |
| 11 | 200 | 100 | 0 |
| 12 | 200 | 100 | 0 |
| Benomyl | 200 | 0 | 100 |
| Thiophanate-methyl | 200 | 0 | 100 |

As understood from the results shown in Table 10, the N-phenylcarbamates (I) of the invention show an excellent preventive effect on the drug-resistant strain but do not show any preventive effect on the tested drug-sensitive strain. On the contrary, commercially available known fungicides such as Benomyl and Thiophanate-methyl show a notable controlling effect on the drug-sensitive strain but not on the drug-resistant strain.

Experiment 8

Preventive effect on rice blast *(Pyricularia oryzae)*:—

Plastic pots of 90 ml were filled with a cultured soil for rice plants, and seeds of rice plant (var: Kinki No. 33) were sowed therein and cultivated in a greenhouse. Onto the seedlings of 3-leaf stage, the test compound formulated in an emulsifiable concentrate and diluted with water was sprayed to enough cover the leaf surfaces with droplets. After air-drying, the seedlings were inoculated with a spore suspension of *Pyricularia oryzae* by spraying, followed by incubation at 28°C for 3 days under a high humid condition. The disease severity were observed and the degree of damage was determined in the same manner as in Experiment 1. The results are shown in Table 11.

Table 11

| Compound No. | Concetration of active ingredient (ppm) | Prevention value (%) |
|---|---|---|
| 11 | 1000 | 95 |
| 12 | 1000 | 99 |
| 13 | 1000 | 97 |
| Edifenphos | 500 | 97 |

Experiment 9

Phytotoxicity to crop plants:—

Plastic pots of 150 ml volume were filled with sandy soil, and seeds of wheat (var: Norin No. 61), apple (var: Kogyoku) and peanut (var: Chiba hanryusei) were sowed therein. Cultivation was carried out in a greenhouse. Onto the resulting seedlings, the test compound formulated in emulsifiable concentrate or wettable powder and diluted with water was sprayed. After cultivation in the greenhouse for additional 10 days, the phytotoxicity was examined on the following criteria:

| Extent | Observation |
|---|---|
| − | No abnormality |
| + | Abnormality due to phytotoxicity observed in a part of crop plants |
| ++ | Abnormality due to phytotoxicity observed in entire crop plants |
| +++ | Crop plants withered due to phytotoxicity |

The results are shown in Table-12.

Table 12

| Compound No. | Concentration of active ingredient (ppm) | Phytotoxicity | | |
|---|---|---|---|---|
| | | Wheat | Apple | Peanut |
| 9 | 1000 | – | – | – |
| 13 | 1000 | – | – | – |
| 14 | 1000 | – | – | – |
| Barban | 1000 | – | ++ | ++ |
| CEPC | 1000 | – | ++ | ++ |
| Swep | 1000 | ++ | ++ | + |

As understood from the results shown in Table 12, the N-phenylcarbamates (I) of the invention produce no material phytotoxicity, while commercially available herbicides having a chemical structure similar thereto produce considerable phytotoxicity.

Experiment 10

Preventive effect on powdery mildew of cucumber *(Sphaerotheca fuliginea)*:—

A plastic pot of 90 ml volume was filled with sandy soil, and seeds of cucumber (var: Sagami-hanjiro) were sowed therein. Cultivation was carried out in a greenhouse for 8 days. Onto the resulting seedlings having cotyledons, the test compound formulated in emulsifiable concentrate or wettable powder and diluted with water were sprayed at a rate of 10 ml per pot. Then, the seedlings were inoculated with a mixed spore suspension of the drug-resistant and drug-sensitive strain of *Sphaerotheca fuliginea* by spraying and further cultivated in the greenhouse. Ten days thereafter, the infectious state of the plants was observed. The degree of damage was determined in the same manner as an Experiment 1, and the results are shown in Table 13.

Table 13

| Compound No. | Concentration of active ingredient (ppm) | Prevention value (%) |
|---|---|---|
| 7 | 100 | 40 |
| 7 | 20 | 0 |
| 9 | 100 | 42 |
| 9 | 20 | 0 |
| 12 | 100 | 42 |
| 12 | 20 | 0 |
| 13 | 100 | 44 |
| 13 | 20 | 0 |
| 14 | 100 | 42 |
| 14 | 20 | 0 |
| 17 | 100 | 42 |
| 17 | 20 | 0 |
| 22 | 100 | 42 |
| 22 | 20 | 0 |
| 27 | 100 | 36 |
| 27 | 20 | 0 |

continued

| Compound No. | Concentration of active ingredient (ppm) | Prevention value (%) |
|---|---|---|
| A | 100 | 42 |
| A | 20 | 10 |
| B | 500 | 42 |
| B | 100 | 8 |
| C | 100 | 42 |
| C | 20 | 10 |
| D | 500 | 34 |
| D | 100 | 0 |
| E | 100 | 44 |
| E | 20 | 8 |
| F | 100 | 42 |
| F | 20 | 6 |
| G | 100 | 42 |
| G | 20 | 8 |
| H | 100 | 40 |
| H | 20 | 5 |
| 7 + A | 20 + 20 | 100 |
| 7 + F | 20 + 20 | 100 |
| 9 + A | 20 + 20 | 100 |
| 9 + B | 20 + 20 | 100 |
| 9 + C | 20 + 20 | 100 |
| 9 + D | 20 + 20 | 100 |
| 12 + G | 20 + 20 | 100 |
| 12 + H | 20 + 20 | 100 |
| 13 + A | 20 + 20 | 100 |
| 13 + B | 20 + 20 | 100 |
| 13 + E | 20 + 20 | 100 |
| 13 + F | 20 + 20 | 100 |
| 14 + A | 20 + 20 | 100 |
| 14 + E | 20 + 20 | 100 |
| 17 + A | 20 + 20 | 100 |
| 17 + B | 20 + 20 | 100 |
| 22 + G | 20 + 20 | 100 |
| 22 + H | 20 + 20 | 100 |
| 27 + A | 20 + 20 | 100 |
| 27 + B | 20 + 20 | 100 |
| 27 + C | 20 + 20 | 100 |
| 27 + D | 20 + 20 | 100 |

As understood from the results shown in Table 13, the combined use of the N-phenylcarbamates (I) of the invention with benzimidazole, thiophanate and/or cyclic imide fungicides show much more excellent preventive effect than their sole use.

Experiment 11

Preventive effect on gray mold of tomato *(Botrytis cinerea)*:—

A plastic pot of 90 ml volume was filled with sandy soil, and seeds of tomato (var: Fukuji No. 2) were sowed therein. Cultivation was carried out in a greenhouse for 4 weeks. Onto the resulting seedlings at the 4-leaf stage, the test compound(s) formulated in emulsifiable concentrate or wettable powder and diluted with water were sprayed at a rate of 10 ml per pot. Then, the seedlings were inoculated with a mixed spore suspension of the drug-resistant and drug-sensitive strain of *Botrytis cinerea* by spraying and placed at 20°C in a room of high humidity for 5 days. The degree of damage was determined in the same manner as in Experiment 1, and the results are shown in Table 14.

Table 14

| Compound No. | Concentration of active ingredient (ppm) | Prevention value (%) |
|---|---|---|
| 9 | 50 | 42 |
| 9 | 10 | 0 |
| 10 | 50 | 34 |
| 10 | 10 | 0 |
| 13 | 50 | 40 |
| 13 | 10 | 0 |
| 14 | 50 | 44 |
| 14 | 10 | 0 |
| 15 | 50 | 44 |
| 15 | 10 | 0 |
| 22 | 50 | 33 |
| 22 | 10 | 0 |
| 27 | 50 | 27 |
| 27 | 10 | 0 |
| 31 | 50 | 44 |
| 31 | 10 | 0 |
| 42 | 50 | 36 |
| 42 | 10 | 0 |
| I | 100 | 46 |
| I | 20 | 16 |
| J | 100 | 42 |
| J | 20 | 14 |
| K | 100 | 40 |
| K | 20 | 8 |
| L | 100 | 38 |
| L | 20 | 8 |
| 9 + I | 10 + 20 | 100 |
| 9 + J | 10 + 20 | 100 |
| 9 + K | 10 + 20 | 100 |
| 9 + L | 10 + 20 | 100 |

continued

| Compound No. | Concentration of active ingredient (ppm) | Prevention value (%) |
|---|---|---|
| 10 + I | 10 + 10 | 100 |
| 10 + K | 10 + 10 | 100 |
| 13 + I | 10 + 10 | 100 |
| 13 + L | 10 + 10 | 100 |
| 14 + I | 10 + 20 | 100 |
| 14 + J | 10 + 20 | 100 |
| 15 + I | 10 + 20 | 100 |
| 15 + L | 10 + 20 | 100 |
| 22 + I | 10 + 10 | 100 |
| 22 + J | 10 + 10 | 100 |
| 27 + K | 10 + 10 | 100 |
| 27 + L | 10 + 10 | 100 |
| 31 + I | 10 + 20 | 100 |
| 31 + J | 10 + 20 | 100 |
| 42 + K | 10 + 20 | 100 |
| 42 + L | 10 + 20 | 100 |

As understood from the results shown in Table 14, the combined use of the N-phenylcarbamates (I) of the invention with benzimidazole, thiophanate and/or cyclic imide fungicides show much more excellent preventive effect than their sole use.

**Claims**

1. A fungicidal composition which comprises as an active ingredient a fungicidally effective amount of an N-phenylcarbamate of the formula:

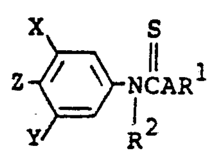

(I)

wherein X and Y are the same or different and each represent a halogen atom, a lower alkyl group, a lower alkenyl group, a lower cyanoalkenyl group, a lower alkynyl group, a lower alkoxy group, a cyano group, a lower alkyl group substituted with at least one halogen atom, hydroxyl or cyano group, or a group of the formula:

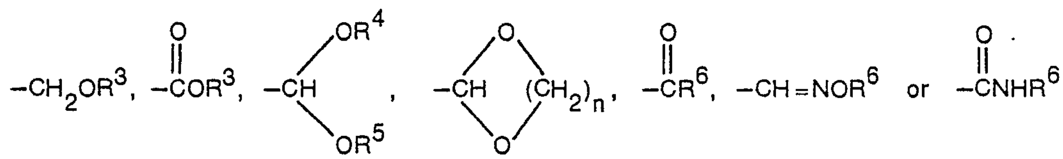

in which $R^3$ is a lower alkyl group, a lower alkenyl group, a lower alkynyl group or a lower haloalkyl group, $R^4$ and and $R^5$ are the same or different and each represent a lower alkyl group, $R^6$ is a hydrogen atom or a lower alkyl group and n is 2, 3 or 4; Z is a hydrogen atom, a fluorine atom or a group of the formula $—OR^7$ in which $R^7$ is a lower alkyl group, a lowewr alkenyl group, a lower alkynyl group or a lower alkyl group substituted with at least one halogen atom, lower alkoxy or lower cycloalkyl group; A is an oxygen atom or a sulfur atom; $R^1$ is a $C_1—C_8$ alkyl group, a $C_3—C_8$ alkenyl group, a $C_3—C_8$ alkynyl group, a lower cycloalkyl

group, a lower haloalkenyl group, a lower haloalkynyl group or a lower alkyl group substituted with at least one halogen atom, cyano, lower alkoxy, lower alkenyloxy, lower haloalkoxy, phenoxy, lower aralkyloxy, phenyl or lower cycloalkyl group; and $R^2$ is a hydrogen atom, a cyano group, a lower alkyl group, a lower alkenyl group, a lower alkynyl group, a lower cycloalkyl group, a lower haloalkenyl group, a lower alkyl group substituted with at least one halogen atom, cyano, lower alkoxy, lower alkoxycarbonyl, lower alkylcarbonyl, phenoxycarbonyl (the phenoxy group optionally being substituted with at least one halogen atom, cyano, nitro, trifluoromethyl, lower alkyl or lower alkoxy group), lower cycloalkyl phenoxy (the pheoxy group optionally being substituted with at least one halogen atom and/or at least one alkyl group) or heteroaryl group (the heteroaryl group optionally being substituted with at least one halogen atom and/or at least one alkyl group), a phenyl group, a phenyl group substituted with at least one halogen atom, cyano, nitro, trifluoromethyl, lower alkyl or lower alkoxy group, an aralkyl group (the aralkyl group optionally being substituted with at least one halogen atom and/or at least one alkyl group) or a group of the formula: $-COR^8$ or $-Si(R^9)_3$ in which $R^8$ is a lower alkyl group, a lower alkenyl group, a lower alkynyl group, a lower cycloalkyl group, a lower haloalkenyl group, a lower alkyl group substituted with at least one halogen atom, cyano, lower alkoxy, lower cycloalkyl or phenoxy group (the phenoxy group optionally being substituted with at least one halogen atom and/or at least one alkyl group), a phenyl group, a phenyl group substituted with at least one halogen atom, cyano, nitro, trifluoromethyl, lower alkyl or lower alkoxy group, or an aralkyl group (the aralkyl group optionally being substituted with at least one halogen atom and/or at least one alkyl group) and $R^9$ is a lower alkyl group, a lower alkoxy group, a halogen atom or a group of the formula: $-SR^{10}$ in which $R^{10}$ is a lower alkyl group, a lower alkoxycarbonyl group or a phenyl group (the phenyl group optionally being substituted with at least one halogen atom, cyano, nitro, trifluoromethyl, lower alkyl or lower alkoxy group), together with an inert carrier or diluent.

2. A fungicidal composition as claimed in claim 1 which further comprises as an additional active ingredient(s) a benzimidazole, thiophanate and/or a cyclic imide fungicide.

3. A fungicidal composition as claimed in claim 2 wherein the benzimidazole fungicide is methyl - 1 - (butylcarbamoyl)benzimidazol - 2 - ylcarbamate, 2 - (2 - furyl) benzimidazole, 2 - (4 - thiazolyl)benzimidazole or methyl benzimidazol-2-ylcarbamate.

4. A fungicidal composition as claimed in claim 2 wherein the thiophanate fungicide is 1,2 - bis(3 - methoxycarbonyl - 2 - thioureido)benzene, 1,2 - bis(3 - ethoxycarbonyl - 2 - thioureido)- benzene, 2 - (O,S - dimethylphosphorylamino) - 1 - (3′ - methoxycarbonyl - 2′ - thioureido)benzene or 2 - (O,O - dimethylthiophosphorylamino) - 1 - (3′ - methoxycarbonyl - 2′ - thioureido)benzene.

5. A fungicidal composition as claimed in claim 2 wherein the cyclic imide fungicide is 3 - (3′,5′ - dichlorophenyl) - 1,2 - dimethylcylcopropane - 1,2 - dicarboximide, 3 - (3′,5′ - dichlorophenyl) - 1 - isopropylcarbamoylimidazolidine - 2,4 - dione, 3 - (3′,5′ - dichlorophenyl) - 5 - methyl - 5 - vinyloxazolidine - 2,4 - dione or ethyl (RS) - 3 - (3′,5′ - dichlorophenyl) - 5 - methyl - 2,4 - dioxooxazolidine - 5 - carboxylate.

6. A N-phenylcarbamate of the formula:

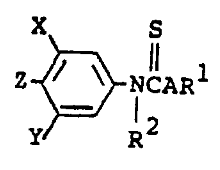

(I)

wherein X, Y, Z, A, $R^1$ and $R^2$ are as defined in claiim 1 with the provisos that when X is a chlorine atom, Z and $R^2$ are each a hydrogen atom, A is an oxygen atom and $R^1$ is an ethyl group, Y can not be a chlorine atom, a methyl group or a methoxy group and that when Z and $R^2$ are each a hydrogen atom, A is an oxygen atom and $R^1$ is an ethyl group, X and Y can not be simultaneously a methyl group or a methoxy group.

7. A method for controlling plant pathogenic fungi which comprises applying a fungicidally effective amount of at least one N-phenylcarbamate of the formula:

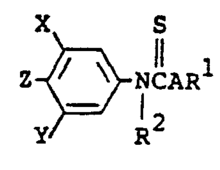

wherein X, Y, Z, A, $R^1$ and $R^2$ are each as defined in claim 1, to plant pathogenic fungi.

8. A method as claimed in claim 7 wherein the plant pathogenic fungi is the drug-resistant strain.

9. A method for controlling plant pathogenic fungi which comprises applying a fungicidally effective amount of a mixture of an N-phenylcarbamate of the formula:

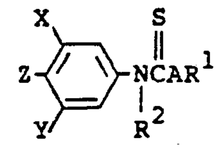

wherein X, Y, Z, A, $R^1$ and $R^2$ are each as defined in claim 1 and a benzimidazole, thiophanate and/or a cyclic imide fungicide.

10. The use of an N-phenylcarbamate of the formula:

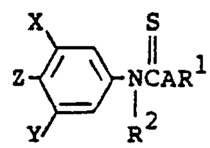

wherein X, Y, Z, A, $R^1$ and $R^2$ are each as defined in claim 1 as a fungicide.

11. A process for producing an N-phenylcarbamate of the formula:

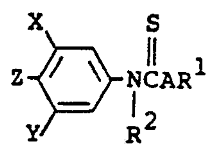

wherein X, Y, Z, A, $R^1$ and $R^2$ are each as defined in claiim 1 with the provisos that when X is a chlorine atom, Z and $R^2$ are each a hydrogen atom, A is an oxygen atom and $R^1$ is an ethyl, group, Y can not be a chlorine atom, a methyl group or a methoxy group and that when Z and $R^2$ are each a hydrogen atom, A is an oxygen atom and $R^1$ is an ethyl group, X and Y can not be simultaneously a methyl group or a methoxy group, which comprises reacting an aniline derivative of the formula:

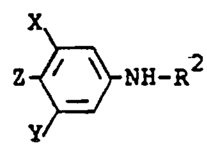

wherein X, Y, Z and $R^2$ are each as defined above, with a chloroformate of the formula:

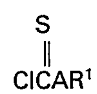

wherein A nad $R^1$ are each as defined above.

12. A process for producing an N-phenylcarbamate of the formula:

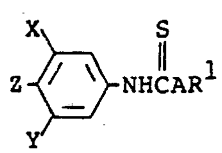

wherein X, Y, Z, A and $R^1$ are each as defined in claim 9, which comprises reacting a phenyl isothiocyanate of the formula:

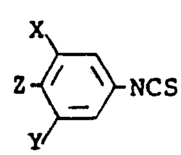

wherein X, Y and Z are each as defined above with an alcohol or a thiol of the formula:

$HAR^1$

wherein A and $R^1$ are each as defined above.

13. A process for producing a N-phenylcarbamate of the formula:

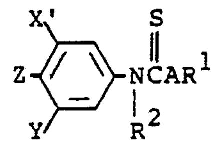

wherein X′ is hydroxyiminomethyl or lower alkoxyiminomethyl and Y, Z, A, $R^1$ and $R^2$ are each as defined in claim 9, which comprises reacting an N-phenylcarbamate of the formula:

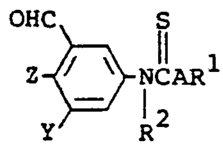

wherein Y, Z, A, $R^1$ and $R^2$ are each as defined above, with hydroxylamine or lower alkoxyamine.

## Patentansprüche

1. Fungizide Zusammensetzung, dadurch gekennzeichnet, daß sie als wirksamen Bestandteil eine fungizid wirkende Menge eines N-Phenylcarbamates der Formel

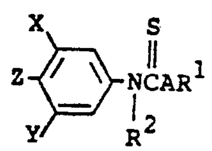

(I)

enthält, wobei

X und Y gleich oder verschieden sind und jeder für sich ein Halogenatom, einen niederen Alkylrest, niederen Alkenylrest, niederen Cyanoalkenylrest, niederen Alkinylrest oder niederen Alkoxyrest, eine Cyanogruppe oder einen niederen Alkylrest bedeutet, und letzterer mindestens ein Halogenatom, eine Hydroxyl- oder Cyanogruppe trägt, oder eine Gruppe der Formel

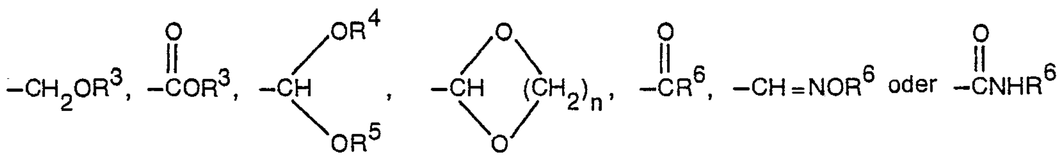

oder

bedeutet, wobei

$R^3$ ein niederer Alkylrest, ein niederer Alkenylrest, ein niederer Alkinylrest oder niederer Halogenalkylrest ist,

$R^4$ und $R^5$ gleich oder verschieden sind und jeder für sich ein niederer Alkylrest ist, und

$R^6$ ein Wasserstoffatom oder ein niederer Alkylrest ist und

n den Wert 2, 3 oder 4 hat,

Z ein Wasserstoff- oder Fluoratom oder eine Gruppe der Formel $-OR^7$ ist, wobei

$R^7$ ein niederer Alkylrest, niederer Alkenylrest oder niederer Alkinylrest oder ein niederer Alkylrest ist, welch letzterer mindestens ein Halogenatom, einen niederen Alkoxyrest oder niederen Cycloalkylrest trägt,

A ein Sauerstoff oder Schwefelatom bedeutet,

$R^1$ einen $C_{1-8}$-Alkylrest, einen $C_{3-8}$-Alkenylrest, einen $C_{3-8}$-Alkinylrest, einen niederen Cycloalkylrest, einen niederen Halogenalkenylrest oder einen niederen Halogenalkinylrest oder einen niederen Alkylrest bedeutet, welch letzterer mindestens ein Halogenatom, eine Cyanogruppe, einen niederen Alkoxyrest, niederen Alkenyloxyrest oder niederen Halogenalkoxyrest, eine Phenoxygruppe, einen niederen Aralkyloxyrest, eine Phenylgruppe oder einen niederen Cycloalkyrest trägt,

$R^2$ ein Wasserstoffatom, eine Cyanogruppe, einen niederen Alkylrest, niederen Alkenylrest, niederen Alkinylrest, niederen Cycloalkylrest oder niederen Halogenalkenylrest oder einen niederen Alkylrest

bedeutet, welch letzterer mindestens ein Halogenatom, eine Cyanogruppe, einen niederen Alkoxyrest, niederen Alkoxycarbonylrest oder niederen Alkylcarbonylrest, eine Phenoxycarbonylgruppe (die Phenoxygruppe trägt gegebenenfalls mindestens ein Halogenatom, eine Cyano-, Nitro- oder Trifluormethylgruppe, einen niederen Alkyl- oder niederen Alkoxyrest), eine niederen Cycloalkylrest, eine Phenoxygruppe (die Phenoxygruppe trägt gegebenenfalls mindestens ein Halogenatom und/oder mindestens einen Alkylrest) oder einen Heteroarylrest (der Heteroarylrest trägt gegebenenfalls mindestens ein Halogenatom und/oder mindestens eine Alkylrest) trägt, oder $R^2$ bedeutet eine Phenylgruppe, eine Phenylgruppe, die mindestens ein Halogenatom, eine Cyano-, Nitro- oder Trifluormethylgruppe, einen niederen Alkly- oder Alkoxyrest trägt, einen Aralkylrest (der Aralkylrest trägt gegebenenfalls mindestens ein Halogenatom und/oder mindestens einen Alkylrest) oder eine Gruppe der Formel —$COR^8$ oder —$Si(R^9)_3$, wobei $R^8$ einen niederen Alkylrest, niederen Alkenylret, niederen Alkinylrest, niederen Cycloälkylrest oder niederen Halogenalkenylrest oder einen niederen Alkylrest bedeutet, wobei letzterer mindestens ein Halogenatom, eine Cyanogruppe, einen niederen Alkoxy- oder niederen Cycloalkylrest oder eine Phenoxygruppe (die Phenoxygruppe trägt gegebenenfalls mindestens ein Halogenatom und/ oder mindestens einen Alkylrest) trägt, oder eine Phenygruppe oder eine Phenylgruppe, die mindestens ein Halogenatom, eine Cyano-, Nitro- oder Trifluormethylgruppe, einen niederen Alkylrest oder niederen Alkoxyrest trägt, oder einen Aralkylrest (der Aralkylrest trägt gegebenenfalls mindestens ein Halogenatom und/oder mindestens einen Alkylrest) beudeutet, und $R^9$ einen niederen Alkylrest oder niederen Alkoxyrest oder ein Halogenatom oder eine Gruppe der Formel —$SR^{10}$ bedeutet, wobei $R^{10}$ ein niederer Alkylrest oder niederer Alkoxycarbonylrest oder eine Phenylgruppe ist (die Phenylgruppe trägt gegebenenfalls mindestens ein Halogenatom, eine Cyano-, Nitro- oder Trifluormethylgruppe, einen niederen Alkyl- oder Alkoxyrest), zusammen mit einem inerten Trägerstoff oder einem Verdünnungsmittel.

2. Fungizide Zusammensetzung gemäß Anspruch 1, gekennzeichnet durch den Zusatz eines weiteren aktiven fungiziden Bestandteils auf Basis eines Benzimidazols, Thiophanates und/oder cylischen Imids.

3. Fungizide Zusammensetzung gemäß Anspruch 2, in welcher das fungizide Benzimidazol 1-(Butylcarbamoyl) - benzimidazol - 2 - ylcarbaminsäure - methylester, 2 - (2 - Furyl) - benzimidazol, 2 - (4-Thiazolyl) - benzimidazol oder Benzimidazol - 2 - ylcarbaminsäuremethylester ist.

4. Fungizide Zusammensetzung gemäß Anspruch 2, in welcher das fungizide Thiophanat 1,2-Bis - (3 - methoxycarbonyl - 2 - thioureido) - benzol, 1,2 - Bis - (3 - äthoxycarbonyl - 2 - thioureido) - benzol, 2 - (O,S - Dimethylphosphorylamino) - 1 - (3' - methoxycarbonyl - 2' - thioureido) - benzol oder 2 - (O,O - Dimethylthiophosphorylamino) - 1 - (3' - methoxycarbonyl - 2' - thioureido) - benzol ist.

5. Fungizide Zusammensetzung gemäß Anspruch 2, in welcher das fungizide cyclishe Imid 3 - (3',5' - Dichlorphenyl) - 1,2 - dimethyl - cyclopropan - 1,2 - dicarboximid, 3 - (3',5' - Dichlorphenyl) - 1 - isopropyl - carbamoylimidazolidin - 2,4 - dion, 3 - (3',5' - Dichlorphenyl) - 5 - methyl - 5 - vinyloxazolidin - 2,4 - dion oder (RS) - 3 - (3',5' - Dichlorphenyl) - 5 - methyl - 2,4 - dioxooxazolidin - 5 - carboxyäthylester ist.

6. N-Phenylcarbamat der Formel

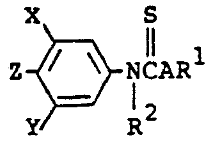

(I)

in der X, Y, Z, A, $R^1$ und $R^2$ die in Anspruch 1 angegebene Bedeutung haben, mit der Maßgabe, daß, wenn X ein Chlor- atom, Z und $R^2$ beides Wasserstoffatome, A ein Sauerstoffatom und $R^1$ eine Äthylgruppe sind, dann Y weder ein Chloratom noch eine Methyl- oder Methoxygruppe sein kann, und daß, wenn Z und $R^2$ beides Wasserstoffatome sind, und A ein Sauerstoffatom und $R^1$ eine Äthylgruppe bedeuten, dann X bzw. Y nicht gleichzeitig eine Methyl- oder Methoxygruppe sein können.

7. Verfahren zur Bekämpfung pflanzenpathogener Pilze, dadurch gekennzeichnet, daß man eine fungizid wirkende Menge mindestens eines N-Phenylcarbamates der Formel

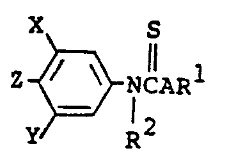

in der X, Y, Z, A, $R^1$ und $R^2$ die in Anspruch 1 angegebene Bedeutung haben, gegen die pflanzenpathogenen Pilze zur Anwendung bringt.

8. Verfahren gemäß Anspruch 7, in welcher der pflaznenpathogene Pilz ein Wirkstoff-resistenter Stamm ist.

9. Verfahren zur Bekämpfung pflanzenpathogener Pilze, dadurch gekennzeichnet, daß man fungizid wirkende Mengen einer Mischung eines N-Phenylcarbamats der Formel

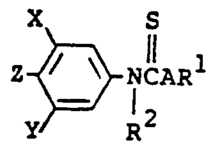

in der X, Y, Z, A, $R^1$ und $R^2$ die in Anpsruch 1 angebenen Bedeutung haben, mit Fungiziden auf Basis von Benzimidazol, Thiophanat und/oder cylischen Imiden zur Anwendung bringt.

10. Verwendung von N-Phenylcarbamaten der Formel

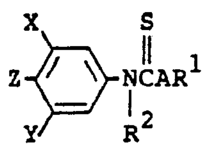

in der X, Y, Z, A, $R^1$ und $R^2$ die in Anspruch 1 angegebene Bedeutung haben, als Fungizid.

11. Verfahren zur Herstellung von N-Phenylcarbamaten der Formel

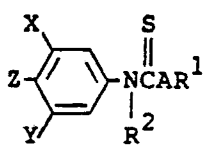

in der X, Y, Z, A, $R^1$ und $R^2$ die in Anspruch 1 angegebene Bedeutung haben, mit der Maßgabe, daß, wenn X ein Chloratom, Z und $R^2$ beides Wasserstoffatome, A ein Sauerstoffatom und $R^1$ eine Äthylgruppe sind, dann Y weder Chlor, noch eine Methyl- oder Methoxygruppe sein kann, und daß, wenn Z und $R^2$ beides Wasserstoffatome sind und A ein Sauerstoffatom und $R^1$ eine Äthylgruppe bedeuten, dann X und Y nicht gleichzeitig eine Methyl- oder Methoxygruppe sein können, dadurch gekennzeichnet, daß man ein Anilinderivat der Formel

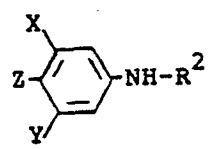

in der X, Y, Z und $R^2$ die vorstehende Bedeutung haben, mit einem Chlorameisensäure-Derivat der Formel

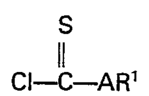

in der A und $R^1$ die vorstehende Bedeutung haben, umsetzt.

12. Verfahren zur Herstellung von N-Phenylcarbamaten der Formel

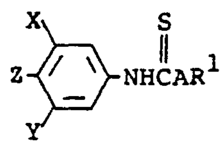

in der X, Y, Z, A und $R^1$ die in Anspruch 9 angegebene Bedeutung haben, dadurch gekennzeichnet, daß man ein Phenylisothiocyanat der Formel

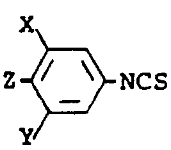

in der X, Y und Z die vorstehende Bedeutung haben, mit einem Alkohol oder einem Mercaptan der Formel

$HAR^1$,

in der A und $R^1$ vorstehende Bedeutung haben, unsetzt.

13. Verfahren zur Herstellung von N-Phenylcarbamaten der Formel

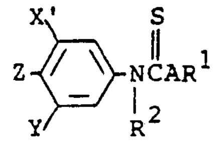

in der X' eine Hydroxyiminomethylgruppe oder einen niedeeren Akoxyiminomethylrest bedeutet und in der Y, Z, A, $R^1$ und $R^2$ die in Anspruch 9 angegebene Bedeutung haben, dadurch gekennzeichnet, daß man ein N-Phenylcarbamat der Formel

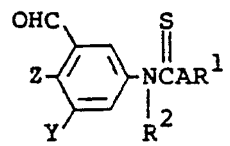

in der Y, Z, A, $R^1$ und $R^2$ die vorstehende Bedeutung haben, mit Hydroxylamin oder einem niederen Alkoxyamin umsetzt.

**Revendications**

1. Composition fongicide caractérisée en ce qu'elle contient, à titre d'ingrédient actif, une quantité efficace du point de vue fongicide d'un N-phénylcarbamate de la formule:

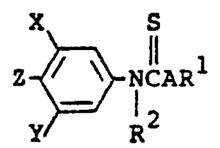

(I)

dans laquelle X et Y sont identiques ou différents et représentent chacun un atome d'halogène, un radical alkyle inférieur, un radical alcényle inférieur, un radical cycloalcényle inférieur, un radical alcynyle inférieur, un radical alcoxy inférieur, un radical cyano, un radical alkyle inférieur substitué par au moins un atome d'halogène, un radical hydroxyle ou cyano ou un radical de la formule:

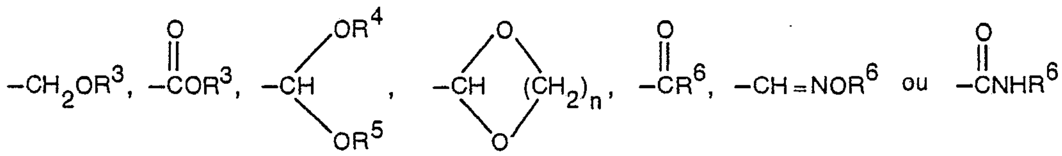

où $R^3$ représente un radical alkyle inférieur, un radical alcényle inférieur, un radical alcynyle inférieur ou un radical haloalkyle inférieur, $R^4$ et $R^5$ sont identiques ou différents et représentent chacun un radical alkyle inférieur, $R^6$ représente un atome d'hydrogène ou un radical alkyle inférieur et n est égal à 2, 3 ou 4; Z représente un atome d'hydrogène, un atome de fluor ou un radical de la formule: $OR^7$ dans lequel $R^7$ représente un radical alkyle inférieur, un radical alcényle inférieur, un radical alcynyle inférieur ou un radical alkyle inférieur substitué par au moins un atome d'halogène, un radical alcoxy inférieur ou cycloalkyle inférieur; A représente un atome d'oxygène ou un atome de soufre; $R^1$ représente un radical alkyle en $C_1$—$C_8$, un radical alcényle en $C_3$—$C_8$, un radical alcynyle en $C_3$—$C_8$, un radical cycloalkyle inférieur, un radical haloalcényle inférieur, un radical haloalcynyle inférieur, ou un radical alkyle inférieur substitué par au moins un atome d'halogène, un radical cyano, alcoxy inférieur, alcényle inférieur oxy, haloalcoxy inférieur, phénoxy, aralkyle inférieur oxy, phényle ou cycloalkyle inférieur; et $R^2$ représente un atome d'hydrogène, un radical cyano, un radical alkyle inférieur, un radical alcényle inférieur, un radical alcynyle inférieur, un radical cycloalkyle inférieur, un radical haloalcényle inférieur, un radical alkyle inférieur substitué par au moins un atome d'halogène, un radical cyano, alcoxy inférieur, alcoxy inférieur carbonyle, alkyle inférieur carbonyle, phénoxycarbonyle (le groupe phénoxy étant éventuellement substitué par au moins un atome d'halogène, un radical cyano, nitro, trifluorométhyle, alkyle inférieur ou alcoxy inférieur), cycloalkyle inférieur, phénboxy (le groupe phénoxy étant éventuellement substitué par au

moins un atome d'halogène et/ou au moins un groupe alkyle) ou hétéroaryle (le groupe hétéroaryle étant éventuellement substitué par au moins un atome d'halogène et/ou au moins un radical alkyle), un radical phényle, un radical phényle substitué par au moins un atome d'halogène, un radical cyano, nitro, trifluorométhyle, alkyle inférieur ou alcoxy inférieur, un radical aralkyle (le groupe aralkyle étant éventuellement substitué par au moins un atome d'halogène et/ou au moins un radical alkyle) ou un radical de la formule: $-COR^8$ ou $-Si(R^9)_3$ où $R^8$ représente un radical alkyle inférieur, un radical alcényle inférieur, un radical alcynyle inférieur, un radical cycloalkyle inférieur, un radical haloalcényle inférieur, un radical alkyle inférieur substitué par au moins un atome d'halogène, un radical cyano, alcoxy inférieur, cycloalkyle inférieur ou phénoxy (le groupe phénoxy étant éventuellement substitué par au moins un atome d'halogène et/ou au moins un groupe alkyle), un radical phényle, un radical phényle substitué par au moins un atome d'halogène, un radical cyano, nitro, trifluorométhyle, alkyle inférieur ou alcoxy inférieur, ou un radical aralkyle (le radical aralkyle étant éventuellement substitué par au moins un atome d'halogène et/ou au moins un radical alkyle) et $R^9$ représente un radical alkyle inférieur, un radical alcoxy inférieur, un atome d'halogène ou un groupe de la formule $-SR^{10}$ dans laquelle $R^{10}$ représente un radical alkyle inférieur, un radical alcoxy inférieur carbonyle, ou un groupe phényle (le radical phényle étant éventuellement substitué par au moins un atome d'halogène, un groupe cyano, nitro, trifluorométhyle, alkyle inférieur ou alcoxy inférieur), ainsi qu'un véhicule ou diluant inerte.

2. Composition fongicide suivant la revendication 1, caractérisée en ce qu'elle comprend également, à titre d'ingrédient(s) actif(s) supplémentaire(s), un fongicide du type benzimidazole, thiophanate et/ou imide cyclique.

3. Composition fongicide suivant la revendication 2, caractérisée en ce que le fongicide du type benzimidazole est le 1-(butylcarbamoyl)benzimidazole - 2 - ylcarbamate de méthyle, le 2 - (2 - furyl) benzimidazole, le 2 - (4 - thiazolyl) - benzimidazole ou le benzimidazole - 2 - ylcarbamate de méthyle.

4. Composition fongicide suivant la revendication 2, caractérisée en ce que le fongicide du type thiophanate est le 1,2 - bis(3 - méthoxycarbonyl - 2 - thiourédio)benzène, le 1,2 - bis(3 - éthoxycarbonyl - 2 - thiouréido) - benzène, le 2 - (O,S - diméthylphosphorylamino) - 1 - (3' - méthoxycarbonyl - 2' - thiouréido)benzène ou le 2 - (O,O - diméthylthiophosphorylamino) - 1 - (3' - méthoxycarbonyl - 2' - thiouréido)benzène.

5. Composition fongicide suivant la revendication 2, caractérisée en ce que le fongicide du type imide cyclique est le 3 - (3',5' - dichlorophényl) - 1,2 - diméthylcylcopropane - 1,2 - dicarboximide, la 3 - (3',5' - dichlorophényl) - 1 - isopropylcarbamoylimidazolidine - 2,4 - dione, la 3 - (3',5'-dichloro - phényl) - 5 - méthyl - 5 - vinyloxazolidine-2,4-dione ou le (RS) - 3 - (3',5'-dichlorophényl)-5-méthyl-2,4-dioxooxazolidine - 5 - carboxylate d'éthyle.

6. N-phénylcarbamate de la formule:

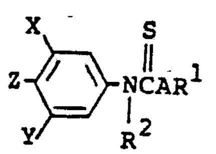

(I)

dans laquelle X, Y, Z, A, $R^1$ et $R^2$ possèdent le mêmes siginifications que celles qui leur ont été attribuées dans la revendication 1, avec les conditions que lorsque X représente un atome de chlore, Z et $R^2$ représentent chacun un atome d'hydrogène, A représente un atome d'oxygène et $R^1$ représente un radical éthyle, Y ne peut être ni un atome de chlore, ni un radical méthyle, ni un radical méthoxy et que lorsque Z et $R^2$ représentent chacun un atome d'hydrogène. A représente un atome d'oxygène et $R^1$ représente un groupe éthyle, X et Y ne représentent pas simultanément un radical méthyle ou un radical éthoxy.

7. Procédé pour combattre des champignons phytopathogènes, caractérisé en ce que l'on applique aux champignons phytopathogènes, une quantité efficace du point de vue fongicide d'au moins un N-phénylcarbamate de la formule:

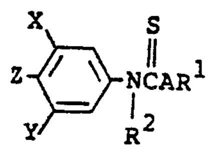

dans laquelle X, Y, Z, A, $R^1$ et $R^2$ possèdent chacun les significations que leur ont été précédemment attribuées dans la revendication 1.

8. Procédé suivant la revendication 7, caractérisé en ce que le champignon phytopathogène est une souche résistant aux produits de traitement.

9. Procédé pour combattre des champignons phytopathogènes, caractérisé en ce que l'on applique une quantité efficace ud point de vue fongicie d'un mélange d'un N-phénylcarbamate de la formule:

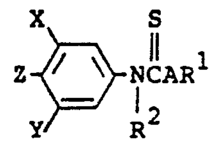

dans laquelle X, Y, Z, A, $R^1$ et $R^2$ possèdent chacun les significations qui leur ont été précedemment attribuées dans la revendication 1 et d'un fongicide du type benzimidazole, thiophanate et/ou imide cyclique.

10. Utilisation d'un N-phénylcarbamate de la formule:

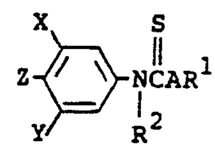

dans laquelle X, Y, Z, A, $R^1$ et $R^2$ possèdent chacun les significations qui leur ont été précédemment attribuées dans la revendication 1, à titre de fongicide.

11. Procédé de production d'un N-phénylcarbamate de la formule:

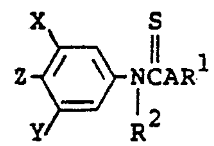

dans laquelle X, Y, Z, A, $R^1$ et $R^2$ possèdent chacun les significations qui leur ont été précédemment attribuées dans la revendication 1, avec les conditions que lorsque X représente un atome de chlore, Z et $R^2$ désignent chacun un atome d'hydrogène, A représente un atome d'oxygène et $R^1$ représente un radical éthyle, Y ne représente ni un atome de chlore, ni un groupe méthyle ni un groupe méthoxy et que lorsque Z et $R^2$ représentent chacun un atome d'hydrogène, A représente un atome d'oxygène et $R^1$ représente un radical éthyle, X et Y ne représentent pas simultanément un radical méthyle ou un radical éthoxy, caractérisé en ce que l'on fait réagir un dérivé de l'aniline de la formule:

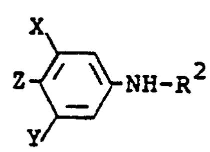

dans laquelle X, Y, Z, et $R^2$ possèdent chacun les significations qui leur ont été précédemment attribuées, sur un chloroformiate de la formule:

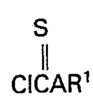

dans laquelle A et $R^1$ possèdent chacun les significations qui leur ont été précédemment attribuées.

12. Procédé de production d'un N-phénylcarbamate de la formule:

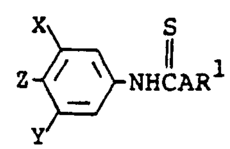

dans laquelle X, Y, Z, A et $R^1$ possèdent chacun les significations qui leur ont été précédemment attribuées dans la revendication 9, caractérisé en ce que l'on fait réagir un isothiocyanate de phényle de la formule:

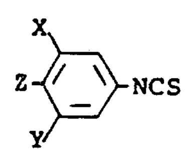

dans laquelle X, Y et Z possèdent chacun les significations qui leur ont été précédemment attribuées, sur un alcool ou un thiol de la formule:

$$HAR^1$$

dans laquelle A et $R^1$ possèdent les signifations que leur ont été précédemment attributées.

13. Procédé de production d'un N-phénylcarbamate de la formule:

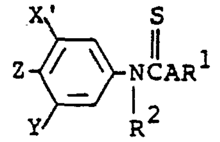

dans laquelle X′ représente un radical hydroxyiminométhyle ou alcoxy inférieur iminométhyle et Y, Z, A, $R^1$ et $R^2$ possèdent chacun les significations qui leur ont été précédemment attribuées dans la revendication 9, caractérisé en ce que l'on fait réagir un N-phénylcarbamate de la formule:

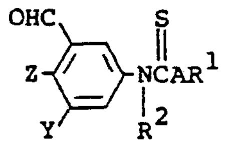

dans laquelle Y, Z, A, $R^1$ et $R^2$ possèdent chacun les significations qui leur ont été précédemment attribuées, sur de l'hydroxylamine ou une alcoxy inférieur amine.